(19) **Europäisches Patentamt European Patent Office Office européen des brevets**

(11) **EP 4 644 420 A1**

(12) **EUROPEAN PATENT APPLICATION**
published in accordance with Art. 153(4) EPC

(43) Date of publication:
05.11.2025 Bulletin 2025/45

(21) Application number: 23910632.1

(22) Date of filing: 26.12.2023

(51) International Patent Classification (IPC):
C07K 16/28 (2006.01)    A61K 39/395 (2006.01)
A61P 35/00 (2006.01)    A61P 37/02 (2006.01)
A61P 31/04 (2006.01)    A61P 31/12 (2006.01)
A61P 33/00 (2006.01)    A61P 19/08 (2006.01)
A61P 37/06 (2006.01)    C07K 19/00 (2006.01)
C12N 15/13 (2006.01)    C12N 15/867 (2006.01)
C12N 5/10 (2006.01)    C12N 15/62 (2006.01)
G01N 33/68 (2006.01)    G01N 33/574 (2006.01)

(86) International application number:
PCT/CN2023/142028

(87) International publication number:
WO 2024/140709 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 26.12.2022 CN 202211681398

(71) Applicant: Unicet Biotech. LLC
Beijing 102206 (CN)

(72) Inventors:
• MA, Weiwei
  Beijing 102206 (CN)
• SUI, Yinqiang
  Beijing 102206 (CN)

(74) Representative: Gille Hrabal
Partnerschaftsgesellschaft mbB
Patentanwälte
Brucknerstraße 20
40593 Düsseldorf (DE)

Remarks:
The complete document including Reference
Table(s) and the Sequence Listing(s) can be
downloaded from the EPO website

(54) **ANTIBODY OR ANTIBODY FRAGMENT TARGETING B7-H3, AND USE THEREOF IN FIELD OF CHIMERIC ANTIGEN RECEPTOR IMMUNE CELL THERAPY**

(57) An antibody or antibody fragment targeting B7-H3, a chimeric antigen receptor based on the antibody or antibody fragment and a chimeric antigen receptor-immune cell, and the use thereof in the treatment of tumors. Specifically, the antibody or antibody fragment comprises a heavy chain variable region VH and a light chain variable region VL, wherein the VH comprises: VH-CDR1 as shown in SEQ ID NO: 9, VH-CDR2 as shown in SEQ ID NO: 10 and VH-CDR3 as shown in SEQ ID NO: 11, and the VL comprises: VL-CDR1 as shown in SEQ ID NO: 12, VL-CDR2 as shown in SEQ ID NO: 13 and VL-CDR3 as shown in SEQ ID NO: 14; or the VH comprises VH-CDR1 as shown in SEQ ID NO: 15, VH-CDR2 as shown in SEQ ID NO: 16 and VH-CDR3 as shown in SEQ ID NO: 17, and the VL comprises: VL-CDR1 as shown in SEQ ID NO: 18, VL-CDR2 as shown in SEQ ID NO: 19 and VL-CDR3 as shown in SEQ ID NO: 20.

FIG. 8

**Description**

The present application claims priority to the prior application with the patent application No. 202211681398.0, entitled "ANTIBODY OR ANTIBODY FRAGMENT TARGETING B7-H3, AND USE THEREOF IN FIELD OF CHIMERIC ANTIGEN RECEPTOR IMMUNE CELL THERAPY" and filed with the China National Intellectual Property Administration on December 26, 2022, which is incorporated herein by reference in its entirety.

TECHNICAL FIELD

The present disclosure relates to a B7-H3-targeting antibody or antibody fragment, a chimeric antigen receptor (CAR) and a CAR-immune cell based on the antibody or antibody fragment, and use thereof for treating tumors.

BACKGROUND

As an immunotherapy, monoclonal antibodies have demonstrated tremendous therapeutic potential in treating malignant diseases and have received widespread attention. Breakthrough applications of, for example, anti-PD-1 and anti-PD-L1 antibody therapies targeting the B7-H1 (PD-L1)/PD-1 signaling pathway have been achieved in the field of tumor therapy. The success of a monoclonal antibody therapy hinges on the following key factors: selecting a suitable target or immune checkpoint, then reactivating, and enhancing the subject's own immune response.

B7-H3, also known as CD276, is a type I transmembrane protein consisting of 316 amino acids. In humans, based on extramembranous domain differences, B7-H3 can be divided into two different forms: 2Ig-B7-H3 and 4Ig-B7-H3. The extramembranous domain of 2Ig-B7-H3 contains a single pair of immunoglobulin variable (IgV)-like and immunoglobulin constant (IgC)-like domains (IgV- and IgC-domains). The extramembranous segment of 4Ig-B7-H3 contains two pairs of IgV- and IgC-domains.

As a member of the B7 protein family, B7-H3 is not expressed or lowly expressed in normal tissues. However, B7-H3 is expressed in a variety of tumor tissues (more than 60% of tumors express B7-H3), including pancreatic cancer (a 77.8% positivity rate), colorectal cancer (a 63.8% positivity rate), gastric cancer (a 69.2% positivity rate), lung cancer (a 69.5% positivity rate), prostate cancer (a 93% positivity rate), ovarian cancer (a 73.1% positivity rate), etc., and highly expressed in a variety of rare tumors. That is why it was referred to as a "tumor-associated antigen". Therefore, B7-H3 is a pan-tumor universal drug target with enormous potential[1].

Like other members of the B7 protein family such as PD-L1, etc., B7-H3 also functions as an immune check inhibiting molecule. Using neutralizing monoclonal antibodies (mAbs) to block the binding of B7-H3 can enhance the killing functions of cells including NK cells and CD8 killer T cells, promote their infiltration into tumor cells, reduce tumor burden, and improve the survival of tumor-bearing mice[2].

Therefore, given the widespread high expression of B7-H3 in tumor tissues and its low expression in healthy tissues, coupled with its function in the tumor microenvironment, B7-H3 has attracted significant attention for its potential in clinical treatment. A number of clinical trials targeting B7-H3 are already underway. Among these, over 20 clinical trials are based on mAbs, including neutralizing antibodies, antibody-drug conjugates (ADCs), bispecific antibodies, antibody-dependent cell-mediated cytotoxicity (ADCC), NK cell engager-linked antibodies, etc.

Neutralizing antibodies can be used to block ligand-receptor binding, thereby blocking signal transduction and further affecting related cellular functions. Using neutralizing antibodies to block B7-H3 signaling can restore the functionality of immune cells affected by B7-H3 signaling, enhancing their anti-tumor abilities. The therapeutic potential of this approach has been demonstrated in various solid tumors, including ovarian cancer, melanoma, and colorectal cancer[3-5].

ADCs are agents in which an antibody that specifically recognizes an antigen is conjugated to a cytotoxic small-molecule drug. They feature good specificity, good safety profiles, and efficient killing of tumor cells. A research team has validated the effectiveness of a B7-H3-targeting ADC drug in a variety of solid tumor models and its safety profile in primates[6].

Bispecific antibodies combine two antibodies that target different targets. Clinically, a bispecific antibody targeting both B7-H3 and CD3 has been approved for the treatment of solid tumors (clinical trial No: NCT03406949). This bispecific antibody can recruit activated T cells to B7-H3-positive tumor tissues, helping T cells recognize and kill tumor cells.

There has also been progress in treating solid tumors with B7-H3-targeting ADCC. A research team designed a B7-H3-targeting monoclonal antibody with an engineered Fc, and the antibody was shown to be able to enhance the killing of B7-H3-positive tumor cells by killer immune cells through Fc receptors. Its safety profile was also validated in primates[7].

Linking a B7-H3-targeting antibody to an NK cell receptor (CD16) antibody can promote the recognition and killing of B7-H3-positive tumor cells by NK cells. A team further engineered the structure by linking an interleukin-15 (IL15) protein, which can further promote the activity of NK cells, on the basis of a CD16 antibody and a B7-H3 antibody. This antibody-protein complex demonstrated very good effects *in vitro* and in the treatment of solid tumors in model animals[8].

In addition, there has also been progress in treating solid tumors, such as brain glioma[9], atypical teratoid rhabdoid tumor[10], and anaplastic meningioma[11], with B7-H3-targeting chimeric antigen receptor T (CAR-T) cell immunotherapies.

Although multiple B7-H3-targeting immunotherapies (including B7-H3 monoclonal antibodies, CAR-B7-H3-T therapies, etc.) have been developed globally and have achieved some early positive therapeutic effects, immunotherapies of this target still have limitations such as a small selection of antibodies, limited clinical therapeutic effects, etc. There remains a need to further explore new antibodies and new therapeutic approaches.

In addition, CAR-T is a revolutionary type of immunotherapy in which genetic modification is performed based on $\alpha\beta$ T cells, and there has been great clinical progress in it over the past decade. To date, six CAR-T therapeutics have been approved by the FDA, and two CAR-T drugs have been approved in China. All eight CAR-T drugs target CD19 or BCMA, are used for treating B-cell-related hematological malignances, and demonstrate high response rates and disease remission rates. However, CAR-T therapy also has many limitations, such as very limited efficacy against solid tumors, inability to achieve allogeneic treatment and off-the-shelf supply, high prices, etc.

$\gamma\delta$ T cells can resolve these problems to some extent. From the viewpoint of cell type, $\gamma\delta$ T cells are T cells expressing $\gamma\delta$ T cell receptors (TCRs). They are a special class of immune cells with the characteristics of both innate immunity and acquired immunity. They recognize antigens and pathogens independently of the major histocompatibility complex (MHC). Thus, they can achieve allogeneic treatment without any modification while avoiding reactions such as GvHD, etc. Many studies have reported that $\gamma\delta$ T cells have a broad-spectrum anti-tumor ability and demonstrate stronger infiltration and anti-tumor effects on solid tumors. The introduction of CAR into $\gamma\delta$ T cells can further enhance their targeting specificity and tumor-killing efficacy[12, 13]. However, there have been no studies or reports on the application of B7-H3 antibodies to CAR-$\gamma\delta$ T.

Reference 1. Kontos, F., et al., B7-H3: An Attractive Target for Antibody-based Immunotherapy. Clin Cancer Res, 2021. 27(5): p. 1227-1235.

Reference 2. Lee, Y.-H., et al., Inhibition of the B7-H3 immune checkpoint limits tumor growth by enhancing cytotoxic lymphocyte function. Cell research, 2017. 27(8): p. 1034-1045.

Reference 3. Cai, D., et al., Tumor-expressed B7-H3 mediates the inhibition of antitumor T-cell functions in ovarian cancer insensitive to PD-1 blockade therapy. Cell Mol Immunol, 2020. 17(3): p. 227-236.

Reference 4. Lee, Y.H., et al., Inhibition of the B7-H3 immune checkpoint limits tumor growth by enhancing cytotoxic lymphocyte function. Cell Res, 2017. 27(8): p. 1034-1045.

Reference 5. Lu, H., et al., B7-H3 inhibits the IFN-gamma-dependent cytotoxicity of Vgamma9Vdelta2 T cells against colon cancer cells. Oncoimmunology, 2020. 9(1): p. 1748991.

Reference 6. Scribner, J.A., et al., Preclinical Development of MGC018, a Duocarmycin-based Antibody-drug Conjugate Targeting B7-H3 for Solid Cancer. Mol Cancer Ther, 2020. 19(11): p. 2235-2244.

Reference 7. Loo, D., et al., Development of an Fc-enhanced anti-B7-H3 monoclonal antibody with potent antitumor activity. Clin Cancer Res, 2012. 18(14): p. 3834-45. Reference 8. Vallera, D.A., et al., NK-Cell-Mediated Targeting of Various Solid Tumors Using a B7-H3 Tri-Specific Killer Engager In Vitro and In Vivo. Cancers, 2020. 12(9).

Reference 9. Tang, X., et al., Administration of B7-H3 targeted chimeric antigen receptor-T cells induce regression of glioblastoma. Signal Transduct Target Ther, 2021. 6(1): p. 125.

Reference 10. Theruvath, J., et al., Locoregionally administered B7-H3-targeted CAR T cells for treatment of atypical teratoid/rhabdoid tumors. Nat Med, 2020. 26(5): p. 712-719.

Reference 11. Tang, X., et al., Bioactivity and safety of B7-H3-targeted chimeric antigen receptor T cells against anaplastic meningioma. Clinical & Translational Immunology, 2020. 9(6): p. e1137.

Reference 12. Ang, W.X., et al., Electroporation of NKG2D RNA CAR Improves V$\gamma$9V$\delta$2 T Cell Responses against Human Solid Tumor Xenografts. Molecular Therapy - Oncolytics, 2020. 17: p. 421-430.

Reference 13. Rozenbaum, M., et al., Gamma-Delta CAR-T Cells Show CAR-Directed and Independent Activity Against Leukemia. Frontiers in Immunology, 2020. 11.

SUMMARY

To address the problems described above, the present disclosure provides a new B7-H3 antibody or antibody fragment and successfully uses the B7-H3 antibody or antibody fragment in the field of CAR-$\alpha\beta$ T/CAR-$\gamma\delta$ T therapy. The present disclosure provides a new option and viability for clinical treatment and detection targeting B7-H3. Furthermore, the present disclosure addresses limitations of CAR immune cell therapy, such as very limited efficacy against solid tumors, inability to achieve allogeneic treatment and off-the-shelf supply, high prices, etc.

In a first aspect, the present disclosure provides an isolated antibody or antibody fragment that specifically binds to B7-H3, comprising a heavy chain variable region VH and a light chain variable region VL, wherein the heavy chain variable region VH described above comprises: a VH-CDR1 represented by the amino acid sequence of SEQ ID NO: 9, a VH-CDR2 represented by the amino acid sequence of SEQ ID NO: 10, and a VH-CDR3 represented by the amino acid sequence of SEQ ID NO: 11; the light chain variable region VL described above comprises: a VL-CDR1 represented by the amino acid sequence of SEQ ID NO: 12, a VL-CDR2 represented by the amino acid sequence of SEQ ID NO: 13, and a VL-CDR3 represented by the amino acid sequence of SEQ ID NO: 14.

SEQ ID NO: 9: NYWIN
SEQ ID NO: 10: RIAPGTISTYYNEKFKG
SEQ ID NO: 11: QDNYFIN
SEQ ID NO: 12: SASSSISSSDLH
SEQ ID NO: 13: GTSNLAS
SEQ ID NO: 14: QQWFSYPFT

In a second aspect, the present disclosure provides an isolated antibody or antibody fragment that specifically binds to B7-H3, comprising a heavy chain variable region VH and a light chain variable region VL, wherein the heavy chain variable region VH described above comprises: a VH-CDR1 represented by the amino acid sequence of SEQ ID NO: 15, a VH-CDR2 represented by the amino acid sequence of SEQ ID NO: 16, and a VH-CDR3 represented by the amino acid sequence of SEQ ID NO: 17; the light chain variable region VL described above comprises: a VL-CDR1 represented by the amino acid sequence of SEQ ID NO: 18, a VL-CDR2 represented by the amino acid sequence of SEQ ID NO: 19, and a VL-CDR3 represented by the amino acid sequence of SEQ ID NO: 20.

SEQ ID NO: 15: RYDMS
SEQ ID NO: 16: TISDDGRHTYDRDSVKG
SEQ ID NO: 17: HRAITTARFDY
SEQ ID NO: 18: KASQDIYSNIG
SEQ ID NO: 19: HGTNLED
SEQ ID NO: 20: LQYVQFPYT

In a third aspect, the present disclosure provides the isolated antibodies or antibody fragments that specifically bind to B7-H3 according to the first or second aspects, wherein the heavy chain variable region VH described above is selected from any one of: the amino acid sequence set forth in SEQ ID NO: 1; an amino acid sequence having 80% or more, or 85% or more, or 88% or more, or 90% or more, or 93% or more, or 95% or more, or 98% or more identity to the amino acid sequence set forth in SEQ ID NO: 1 outside the CDRs; the amino acid sequence set forth in SEQ ID NO: 2; and an amino acid sequence having 80% or more, or 85% or more, or 88% or more, or 90% or more, or 93% or more, or 95% or more, or 98% or more identity to the amino acid sequence set forth in SEQ ID NO: 2 outside the CDRs.

SEQ ID NO: 1

EVQLQQSGAELVKPGASVKLSCKASGYTFTNYWINWIRQRPGQGLEWIGRIA PGTISTYYNEKFKGRATITEDTSTNTAYLQLSSLTSEDTAVYFCARQDNYFIN WGQGTLVTVSS

SEQ ID NO: 2

EFEVQLVESGGGLVKPGGSLKLSCAASGFAFSRYDMSWVRQSPEKRLEWVA TISDDGRHTYDRDSVKGRFTISRDNAKNTLYLQMSSLRSEDTALYYCVRHRA ITTARFDYWGQGTTVTVSSSR

In a fourth aspect, the present disclosure provides the isolated antibody or antibody fragment that specifically binds to B7-H3 according to the first or second aspect, wherein the light chain variable region VL described above is selected from any one of: the amino acid sequence set forth in SEQ ID NO: 3; an amino acid sequence having 80% or more, or 85% or more, or 88% or more, or 90% or more, or 93% or more, or 95% or more, or 98% or more identity to the amino acid sequence set forth in SEQ ID NO: 3 outside the CDRs; the amino acid sequence set forth in SEQ ID NO: 4; and an amino acid sequence having 80% or more, or 85% or more, or 88% or more, or 90% or more, or 93% or more, or 95% or more, or 98% or more identity to the amino acid sequence set forth in SEQ ID NO: 4 outside the CDRs.

SEQ ID NO: 3

DIVLTQSPASLSASLGEKVTITCSASSSISSSDLHWYQQKSGTSPRPWIYGTSN LASGVPPRFSGSGSGTSFSLTISSVEAEDVGTYYCQQWFSYPFTFGTGTKLEIK

SEQ ID NO: 4

GCADIVMTQSPSSLSVSLGDTVSITCKASQDIYSNIGWLQQLPGQSFKGLIYH GTNLEDGVPSRFSGSGSGTDYSLTISGLESEDFADYYCLQYVQFPYTFGGGTK LEIKASG

In a fifth aspect, the present disclosure provides an isolated antibody or antibody fragment that specifically binds to B7-H3, comprising: (i) a heavy chain variable region VH represented by SEQ ID NO: 1 and a light chain variable region VL represented by SEQ ID NO: 3, or (ii) a heavy chain variable region VH represented by SEQ ID NO: 2 and a light chain variable region VL represented by SEQ ID NO: 4.

In a sixth aspect, the present disclosure provides the isolated antibody or antibody fragment that specifically binds to B7-H3 according to any one of the first to fifth aspects described above, wherein the antibody or antibody fragment described above is a genetically engineered antibody or antibody fragment.

In a seventh aspect, the present disclosure provides the isolated antibody or antibody fragment that specifically binds to B7-H3 according to any one of the first to sixth aspects described above, wherein the antibody or antibody fragment is an scFv.

In an eighth aspect, the present disclosure provides a chimeric antigen receptor comprising: the antibody or antibody fragment that specifically binds to B7-H3 according to the seventh aspect described above.

In a ninth aspect, the present disclosure provides the chimeric antigen receptor according to the eighth aspect of the present disclosure, which comprises: the antibody or antibody fragment according to the seventh aspect described above, and a transmembrane region fused to a carboxyl-terminus of the antibody or antibody fragment.

In a tenth aspect, the present disclosure provides the chimeric antigen receptor according to the eighth or ninth aspect described above, which comprises: the antibody or antibody fragment according to the seventh aspect described above, a transmembrane region fused to a carboxyl-terminus of the antibody or antibody fragment, and an immunocompetent cell activation signal transduction region fused to the carboxyl-terminus of the transmembrane region.

In an eleventh aspect, the present disclosure provides the chimeric antigen receptor according to any one of the eighth to tenth aspects described above, which further comprises one or two or more of a membrane protein, a secreted protein, an intracellular protein, a small-molecule drug, and a cytotoxic drug.

In a twelfth aspect, the present disclosure provides a nucleic acid molecule comprising: a nucleotide sequence encoding the antibody or antibody fragment according to any one of the first to seventh aspects described above or the chimeric antigen receptor according to any one of the eighth to eleventh aspects described above.

In a thirteenth aspect, the present disclosure provides the nucleic acid molecule according to the twelfth aspect described above, which comprises: (i) the nucleotide sequence set forth in SEQ ID NO: 5, which encodes the heavy chain variable region, and the nucleotide sequence set forth in SEQ ID NO: 7, which encodes the light chain variable region, or (ii) the nucleotide sequence set forth in SEQ ID NO: 6, which encodes the heavy chain variable region, and the nucleotide sequence set forth in SEQ ID NO: 8, which encodes the light chain variable region.

SEQ ID NO: 5

GAAGTGCAGCTCCAGCAGAGCGGAGCAGAACTGGTGAAGCCAGGAGCCA GCGTGAAGCTGTCTTGCAAGGCCAGCGGCTACACCTTCACCAACTACTGG

ATCAATTGGATCAGACAGAGGCCAGGACAGGGACTCGAGTGGATTGGCA GAATCGCCCCAGGCACCATCAGCACCTACTACAACGAGAAGTTCAAGGG CAGGGCCACCATCACCGAGGATACCAGCACCAACACCGCCTACCTCCAGC TGTCTAGCCTGACAAGCGAGGACACAGCCGTGTACTTTGCGCCAGGCAG GACAACTACTTCATCAATTGGGGCCAGGGAACCCTGGTGACAGTGTCCAG C

SEQ ID NO: 6

GAGTTCGAGGTGCAGCTGGTGGAGAGCGGAGGTGGGCTAGTTAAACCCG GCGGCTCCTTGAAGCTGTCATGCGCTGCTTCTGGGTTTGCGTTCTCCCGCT ACGACATGAGTTGGGTGCGCCAGAGCCCTGAAAAGCGCCTGGAGTGGGT CGCCACCATTAGCGATGACGGCAGACACACCTACGACAGGGATAGCGTA AAGGGTCGCTTCACCATCTCTCGTGACAACGCCAAGAACACGCTTTACCT GCAGATGTCCTCTCTGCGCTCGGAGGACACCGCGCTCTACTACTGCGTGC GACATCGCGCCATCACTACTGCACGGTTCGACTATTGGGGCCAGGGCACC ACAGTCACCGTGTCGTCCTCCCGT

SEQ ID NO: 7

GACATTGTTCTTACACAATCTCCGGCCAGCCTCTCTGCGAGCCTCGGGGA AAAGGTGACAATCACTTGTTCCGCATCATCAAGTATCTCCAGCTCTGACC TGCACTGGTATCAGCAGAAAAGTGGCACCAGTCCGAGGCCGTGGATCTAT GGCACTAGCAATCTTGCGTCAGGAGTCCCACCCCGCTTTAGTGGCAGTGG ATCAGGGACATCATTCAGTCTGACAATCTCTAGCGTCGAGGCGGAGGATG TCGGCACATATTACTGCCAACAGTGGTTTTCTTATCCTTTCACATTTGGTA CGGGCACGAAACTGGAAATAAAA

SEQ ID NO: 8

GGGTGCGCGGACATCGTGATGACCCAGAGTCCGTCGTCTCTGAGCGTCTC GCTCGGCGACACCGTGAGCATCACTTGTAAAGCTTCCCAGGACATCTACT CCAACATCGGTTGGCTACAACAGCTGCCCGGACAGTCCTTCAAGGGCCTG ATTTACCACGGGACCAACCTGGAGGACGGCGTTCCTTCCCGCTTCAGCGG CTCCGGCTCCGGTACAGATTACTCTCTGACCATTTCTGGCCTTGAGAGCGA

AGACTTTGCCGATTACTACTGCCTGCAGTACGTGCAGTTCCCCTATACCTT

CGGCGGTGGCACTAAGTTGGAGATCAAGGCCTCAGGC

In a fourteenth aspect, the present disclosure provides a vector comprising the nucleic acid molecule according to the twelfth or thirteenth aspect described above.

In a fifteenth aspect, the present disclosure provides the vector according to the fourteenth aspect, which is a lentiviral vector, a retroviral vector, an adenoviral vector, an adeno-associated virus vector, or the like.

In a sixteenth aspect, the present disclosure provides a cell comprising the nucleic acid molecule according to the twelfth or thirteenth aspect described above or the vector according to the fourteenth or fifteenth aspect.

In a seventeenth aspect, the present disclosure provides the cell according to the sixteenth aspect, which includes autologous or allogeneic T cells, B cells, NK cells, macrophages, monocytes, dendritic cells, neutrophils, basophils, eosinophils, mast cells, NK-T cells, MAIT cells, hematopoietic stem cells, embryonic stem cells, induced pluripotent stem cells, and erythrocytes, wherein the T cells described above include $\alpha\beta$ T cells, $\gamma\delta$ T cells, and regulatory T cells.

In an eighteenth aspect, the present disclosure provides a pharmaceutical composition comprising: any one or more selected from the antibody or antibody fragment according to any one of the first to seventh aspects described above, the chimeric antigen receptor according to any one of the eighth to eleventh aspects, the nucleic acid molecule according to the twelfth or thirteenth aspect, the vector according to the fourteenth or fifteenth aspect, and the cell according to the sixteenth or seventeenth aspect; or comprising: any one or more selected from the antibody or antibody fragment according to any one of the first to seventh aspects described above, the chimeric antigen receptor according to any one of the eighth to eleventh aspects, the nucleic acid molecule according to the twelfth or thirteenth aspect, the vector according to the fourteenth or fifteenth aspect, and the cell according to the sixteenth or seventeenth aspect, and a pharmaceutically acceptable carrier; or comprising: any one or more selected from the antibody or antibody fragment according to any one of the first to seventh aspects described above, the chimeric antigen receptor according to any one of the eighth to eleventh aspects, the nucleic acid molecule according to the twelfth or thirteenth aspect, the vector according to the fourteenth or fifteenth aspect, and the cell according to the sixteenth or seventeenth aspect, and another pharmaceutically active agent or drug.

In a nineteenth aspect, the present disclosure provides a method of treatment or prevention of B7-H3-positive diseases, comprising administering to a subject a therapeutically effective amount of the antibody or antibody fragment according to any one of the first to seventh aspects described above, the chimeric antigen receptor according to any one of the eighth to eleventh aspects, the nucleic acid molecule according to the twelfth or thirteenth aspect, the vector according to the fourteenth or fifteenth aspect, the cell according to the sixteenth or seventeenth aspect, or the pharmaceutical composition according to the eighteenth aspect.

In a twentieth aspect, the present disclosure provides the method according to the nineteenth aspect, wherein the B7-H3-positive diseases described above include malignant/benign hematological tumors, malignant/benign solid tumors, autoimmune diseases, bacterial infections, viral infections, parasitic infections, abnormal bone growth, allogeneic transplantation, transplant rejection, and the like; the autoimmune diseases include systemic lupus erythematosus, rheumatoid arthritis, multiple sclerosis, Sjogren's syndrome, ankylosing spondylitis, and the like.

In a twenty-first aspect, the present disclosure provides the method according to the twentieth aspect, wherein the diseases include acute myeloid leukemia, chronic myeloid leukemia, acute lymphocytic leukemia, non-Hodgkin lymphoma, multiple myeloma, melanoma, lung cancer, colorectal cancer, renal tumors, bladder cancer, gastrointestinal cancer, prostate cancer, liver cancer, ovarian cancer, pancreatic cancer, endometrial cancer, gastric cancer, prostate cancer, kidney cancer, cervical cancer, thyroid cancer, uterine cancer, neuroendocrine cancer, head and neck cancer, nasopharyngeal carcinoma, testicular cancer, basal cell skin cancer, squamous cell skin cancer, dermatofibrosarcoma protuberans, Merkel cell carcinoma, glioblastoma, glioma, sarcomas, mesothelioma, myelodysplastic syndrome, or the like.

In a twenty-second aspect, the present disclosure provides the antibody or antibody fragment according to any one of the first to seventh aspects described above, the chimeric antigen receptor according to any one of the eighth to eleventh aspects described above, or the nucleic acid molecule according to the twelfth or thirteenth aspect described above, the vector according to the fourteenth or fifteenth aspect described above, the cell according to the sixteenth or seventeenth aspect described above, or the pharmaceutical composition according to the eighteenth aspect described above for use in the treatment or prevention of B7-H3-positive diseases.

In a twenty-third aspect, the present disclosure provides the antibody or antibody fragment, the chimeric antigen receptor, the nucleic acid molecule, the vector, the cell, or the pharmaceutical composition for use in the treatment or prevention of B7-H3-positive diseases according to the twenty-second aspect, wherein the B7-H3-positive diseases include malignant/benign hematological tumors, malignant/benign solid tumors, autoimmune diseases, bacterial infections, viral infections, parasitic infections, abnormal bone growth, allogeneic transplantation, and transplant rejection; the autoimmune diseases include systemic lupus erythematosus, rheumatoid arthritis, multiple sclerosis, Sjogren's syndrome, and ankylosing spondylitis.

In a twenty-fourth aspect, the present disclosure provides the antibody or antibody fragment, the chimeric antigen receptor, the nucleic acid molecule, the vector, the cell, or the pharmaceutical composition for use in the treatment or prevention of B7-H3-positive diseases according to the twenty-third aspect, wherein the B7-H3-positive diseases include acute myeloid leukemia, chronic myeloid leukemia, acute lymphocytic leukemia, non-Hodgkin lymphoma, multiple myeloma, melanoma, lung cancer, colorectal cancer, renal tumors, bladder cancer, gastrointestinal cancer, prostate cancer, liver cancer, ovarian cancer, pancreatic cancer, endometrial cancer, gastric cancer, prostate cancer, kidney cancer, cervical cancer, thyroid cancer, uterine cancer, neuroendocrine cancer, head and neck cancer, nasopharyngeal carcinoma, testicular cancer, basal cell skin cancer, squamous cell skin cancer, dermatofibrosarcoma protuberans, Merkel cell carcinoma, glioblastoma, glioma, sarcomas, mesothelioma, or myelodysplastic syndrome.

In a twenty-fifth aspect, the present disclosure provides a method for detecting B7-H3, comprising: a step of using the antibody or antibody fragment according to any one of the first to seventh aspects described above, the chimeric antigen receptor according to any one of the eighth to eleventh aspects described above, or the nucleic acid molecule according to the twelfth or thirteenth aspect described above to detect B7-H3.

In a twenty-sixth aspect, the present disclosure provides a kit for detection of B7-H3, comprising the antibody or antibody fragment according to any one of the first to seventh aspects described above or a labeled form thereof, the chimeric antigen receptor according to any one of the eighth to eleventh aspects described above or a labeled form thereof, or the nucleic acid molecule according to the twelfth or thirteenth aspect described above or a labeled form thereof.

In a twenty-seventh aspect, the present disclosure provides an isolation kit for isolating B7-H3-positive cells, comprising the antibody or antibody fragment according to any one of the first to seventh aspects described above or a labeled form thereof, the chimeric antigen receptor according to any one of the eighth to eleventh aspects described above or a labeled form thereof, or the nucleic acid molecule according to the twelfth or thirteenth aspect described above or a labeled form thereof.

In a twenty-eighth aspect, the present disclosure provides a method for enhancing cell function *in vitro*, comprising: a step of contacting cells with the antibody or antibody fragment according to any one of the first to seventh aspects described above or a labeled form thereof, the chimeric antigen receptor according to any one of the eighth to eleventh aspects described above or a labeled form thereof, or the nucleic acid molecule according to the twelfth or thirteenth aspect described above or a labeled form thereof.

In a twenty-ninth aspect, the present disclosure provides use of the antibody or antibody fragment according to any one of the first to seventh aspects described above for the manufacturing of a product for detecting B7-H3 protein.

In a thirtieth aspect, the present disclosure provides use of the antibody or antibody fragment according to any one of the first to seventh aspects described above for the manufacturing of a product for blocking B7-H3 protein.

BRIEF DESCRIPTION OF THE DRAWINGS

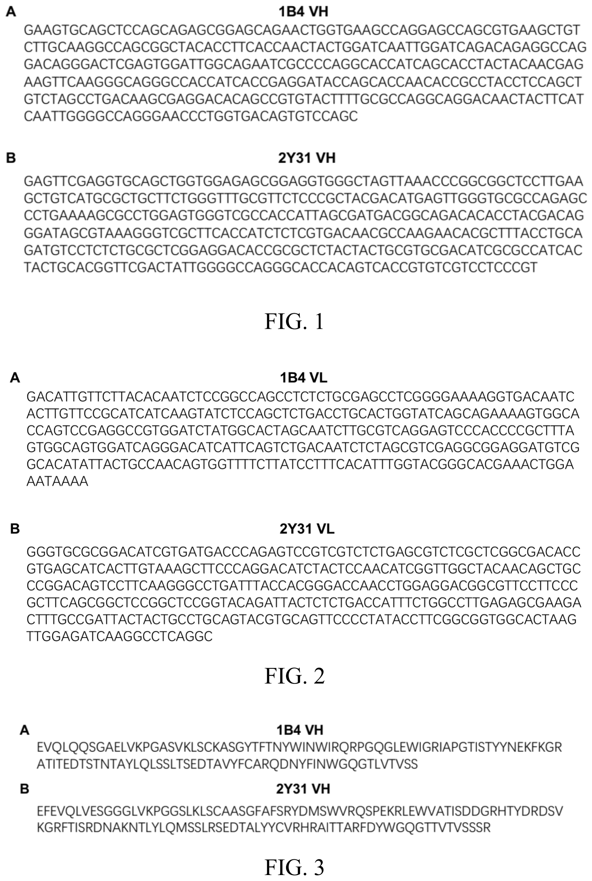

FIG. 1 shows the base sequences of the heavy chain variable regions of antibodies 1B4 and 2Y31, wherein FIG. 1A shows the base sequence of the heavy chain variable region of antibody 1B4 (SEQ ID NO: 5), and FIG. 1B shows the base sequence of the heavy chain variable region of antibody 2Y31 (SEQ ID NO: 6).

FIG. 2 shows the base sequences of the light chain variable regions of antibodies 1B4 and 2Y31, wherein FIG. 2A shows the base sequence of the light chain variable region of antibody 1B4 (SEQ ID NO: 7), and FIG. 2B shows the base sequence of the light chain variable region of antibody 2Y31 (SEQ ID NO: 8).

FIG. 3 shows the amino acid sequences of the heavy chain variable regions of antibodies 1B4 and 2Y31, wherein FIG. 3A shows the amino acid sequence of the heavy chain variable region of antibody 1B4 (SEQ ID NO: 1), and FIG. 3B shows the amino acid sequence of the heavy chain variable region of antibody 2Y31 (SEQ ID NO: 2).

FIG. 4 shows the amino acid sequences of the light chain variable regions of antibodies 1B4 and 2Y31, wherein FIG. 4A shows the amino acid sequence of the light chain variable region of antibody 1B4 (SEQ ID NO: 3), and FIG. 4B shows the amino acid sequence of the light chain variable region of antibody 2Y31 (SEQ ID NO: 4).

FIG. 5 shows affinity assay results for a humanized 1B4 antibody.

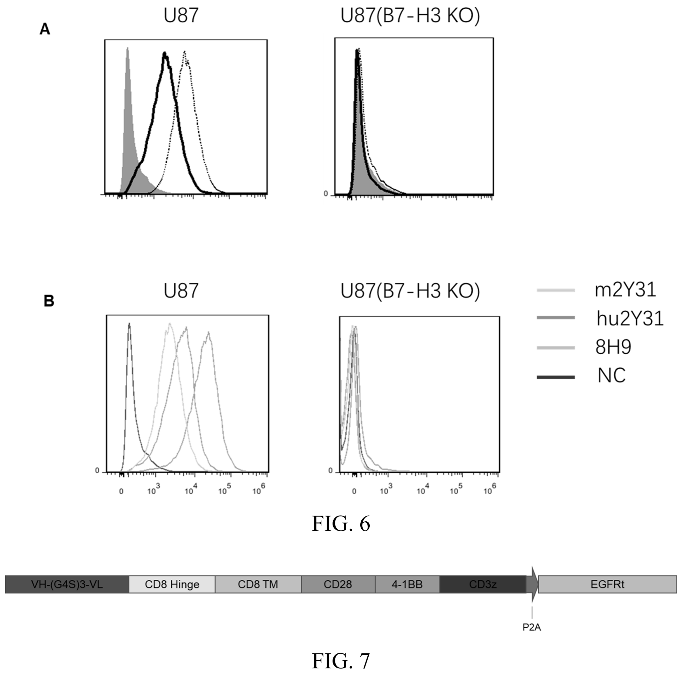

FIG. 6 shows binding results for humanization-engineered antibodies on a U87 glioma cell strain and a B7-H3 knockout U87 cell strain, wherein in FIG. 6A, the gray peaks represent a negative control, the solid-line peaks represent the binding of the parent murine antibody 1B4 on the tumor cell strains, and the dashed-line peaks represent the binding of a humanization-engineered 1B4 antibody on the tumor cell strains; in FIG. 6B, m2Y31 represents the binding of the parent murine antibody 2Y31 to the tumor cell strains, and hu2Y31 represents the binding of a humanized 2Y31 antibody to the tumor cell strains; 8H9 represents the binding of an antibody that recognizes a different antigenic epitope of B7-H3 to the tumor cells, and NC is the negative control.

FIG. 7 is a schematic diagram of the structure of a CAR, wherein an scFv (VH-(G4S)3-VL), a CD8 hinge region (CD8 Hinge), a transmembrane region (CD8 TM), co-stimulatory signals CD28 and 4-1BB, and a CD3z signal activation domain are arranged from the extracellular segment to the intracellular segment, wherein the scFv comprises a heavy chain variable region and a light chain variable region that are linked together by a $(GGGGS)_3$ linker peptide; in addition, at the C-terminus of the CAR structural region, a truncated EGFR structure is linked by a P2A cleavage peptide, wherein the truncated EGFR structure can not only serve as a screening label for CAR-positive cells but also provide a safety switch for clinical studies.

FIG. 8 shows the ability of CAR-B7-H3-expressing Jurkat T cells to bind to B7-H3 protein.

FIG. 9 shows the use of antibody 1B4 for detection: the wild-type RKO colon cancer tumor cells (dark peak, RKO WT) expressed high levels of B7-H3, while the B7-H3 knockout RKO tumor cells (light peak, RKO B7-H3 KO) did not express B7-H3.

FIG. 10 shows expression levels of CD69 after stimulation of CAR-Jurkat T cells constructed using antibody 1B4 by RKO WT or RKO (B7-H3 KO) tumor cells.

FIG. 11A shows the transduction positivity rate of CAR-B7-H3-expressing $\alpha\beta$ T cells (the scFv was the light and heavy chain sequences of antibody 1B4).

FIG. 11B shows expression levels of CD69 after stimulation of $\alpha\beta$ T or CAR-B7-H3-$\alpha\beta$ T cells by RKO WT or RKO (B7-H3 KO) tumor cells.

FIG. 12 shows the ability of CAR-B7-H3-$\alpha\beta$ T cells (the scFv was the light and heavy chain sequences of antibody 1B4) to kill RKO tumor cells *in vitro.*

FIG. 13 shows the ability of CAR-B7-H3-$\alpha\beta$ T cells (the scFv was the light and heavy chain sequences of antibody 1B4) to kill glioma cells *in vitro.*

FIG. 14 shows the transduction positivity rate of CAR-B7-H3-expressing $\gamma\delta$ T cells (the scFv was the light and heavy chain sequences of antibody 1B4).

FIG. 15A shows the killing effects of CAR-B7-H3-expressing CAR-$\alpha\beta$ T and CAR-$\gamma\delta$ T on RKO WT tumor cells (the scFv was the light and heavy chain sequences of antibody 1B4).

FIG. 15B shows the killing effects of CAR-B7-H3-expressing CAR-$\alpha\beta$ T and CAR-$\gamma\delta$ T on B7-H3 knockout RKO tumor cells (the scFv was the light and heavy chain sequences of antibody 1B4).

FIG. 16 shows the ability of CAR-B7-H3-expressing CAR-$\gamma\delta$ T to kill B7-H3-positive SKOV3 tumor cells *in vitro* (the scFv was the light and heavy chain sequences of antibody 1B4 or antibody 2Y31).

FIG. 17 shows the *in vivo* anti-tumor effect of CAR-B7-H3-expressing CAR-$\gamma\delta$ T (the scFv was the light and heavy chain sequences of antibody 1B4) on SKOV3.

FIG. 18 shows growth curves of SKOV3 tumors in model mice.

FIG. 19 shows changes in survival curves of SKOV3 tumor model mice.

## DETAILED DESCRIPTION

The present disclosure is further illustrated below through description of specific embodiments with reference to the drawings. However, these are not intended to limit the present disclosure. Various modifications or improvements can be made based on the essential spirit of the present disclosure by those skilled in the art, and all such modifications and improvements fall within the scope of the present disclosure as long as they do not depart from the essential spirit of the present disclosure.

It should be noted that, unless otherwise defined, the technical terms or scientific terms of the specification shall have the ordinary meanings as understood by those skilled in the art to which the present disclosure pertains. In addition, it should be appreciated that the terms used in the present application are for the purpose of describing specific embodiments only and are not intended to be limiting.

The term "specifically bind" in the specification means that the antigen recognition region of the present disclosure does not or substantially does not cross-react with any polypeptide other than the target antigen. The degree of its specificity can be determined by immunological techniques, including but not limited to immunoblotting, immunoaffinity chromatography, flow cytometry, and the like.

The term "antibody" in the specification refers to an immunoglobulin molecule that specifically binds to an antigen. Antibodies may be intact immunoglobulins derived from natural sources or recombinant sources and may be immunoreactive moieties of intact immunoglobulins. Antibodies are typically tetramers of immunoglobulin molecules. The antibodies in the present disclosure may exist in various forms, including, for example, Fab, Fab', F(ab')2, Fv fragments, linear antibodies formed by antibody fragments, scFv antibodies, ADCs, multispecific antibodies, single-chain antibodies, and humanized antibodies.

The term "antibody fragment" in the specification refers to a portion of an intact antibody structure or sequence.

The term "heavy chain" in the specification refers to the larger of the two types of polypeptide chains present in all antibody molecules in naturally occurring conformations.

The term "light chain" in the specification refers to the smaller of the two types of polypeptide chains present in all antibody molecules in naturally occurring conformations.

The term "heavy chain variable region (VH)" in the specification refers to about **110** amino acid residues of a heavy chain that are near the amino-terminus (N-terminus); the composition and arrangement of these amino acid residues vary widely.

The term "light chain variable region (VL)" in the specification refers to about **110** amino acid residues of a light chain that are near the amino-terminus (N-terminus); the composition and arrangement of these amino acid residues vary widely.

The term "CDRs" in the present disclosure means complementarity-determining regions or complementarity determinants, which refer to non-contiguous antigen-binding sites found within the variable regions of heavy and light chain polypeptides. In the present disclosure, the CDRs and FRs in antibody variable regions are annotated using the Kabat numbering scheme.

The term "identity" in the specification refers to the high-proportion matching property of amino acids or nucleotides shown by comparison of a target amino sequence or a target nucleotide sequence with a reference sequence. In the specification, identity can be determined using standard software such as BLAST or FASTA.

In the specification, "80% or more identity" means that the identity is 80% or more and means that the identity is preferably 85% or more, more preferably 88% or more, yet more preferably 90% or more, still more preferably 93% or more, particularly preferably 95% or more, particularly more preferably 98% or more, and most preferably 100%.

The term "genetically engineered antibody or antibody fragment" in the specification refers to an antibody or antibody fragment that has been engineered or modified by genetic engineering. The "genetic engineering" mentioned in this term refers to a technique of integrating genes from different sources by methods such as gene splicing and DNA recombination techniques and the like and expressing the genes. Specific examples include inserting other proteins or protein fragments into an antibody or antibody fragment, or replacing a certain portion of an antibody or antibody fragment with other proteins or protein fragments, or linking different antibodies or antibody fragments to other proteins or protein fragments to form a larger protein complex. "Humanized antibody" is also a "genetically engineered antibody or antibody fragment" and refers to an antibody that is re-expressed after a non-human (such as murine, rabbit, or the like) monoclonal antibody is engineered using gene cloning and DNA recombination techniques. It features a reduction in the immunogenicity of the corresponding murine antibody while retaining the affinity and antigen-binding specificity of the murine antibody. A basic way of achieving humanization is to use human antibody genes to encode the constant regional portion of a non-human antibody or all portions of the antibody. According to different ways of engineering, humanized antibodies include chimeric antibodies, engineered antibodies, fully humanized antibodies, and other types.

The term "constant regions" in the specification refers to regions of an antibody that are near C-termini and whose amino acid sequences are relatively stable. Constant regions include "light chain constant region" and "heavy chain constant region", which refer to a region of an antibody light chain that is near the C-terminus and whose amino acid sequence is relatively stable and a region of an antibody heavy chain that is near the C-terminus and whose amino acid sequence is relatively stable, respectively.

The term "scFv" in the specification refers to an antibody fragment that is a recombinant protein comprising a heavy chain variable region and a light chain variable region that are linked by a linker that associates the two domains to finally form an antigen-binding site. The size of an scFv is generally 1/6 of that of an intact antibody. The scFv is preferably an amino acid sequence encoded by one nucleotide chain. The scFv used in the present disclosure may be further modified using conventional techniques known in the art, such as amino acid deletion, insertion, substitution, addition, and/or recombination, and/or other modification methods, either alone or in combination. Methods for introducing such modifications into the DNA sequence of an antibody based on its amino acid sequence are well known to those skilled in the art (see, e.g., Sambrook Molecular Cloning: A Laboratory Manual, Cold Spring Harbor Laboratory (1989) N. Y.). The modifications are preferably performed at a nucleic acid level. The scFv described above may also include derivatives thereof. The scFv can be expressed as a single-chain polypeptide. The scFv retains the specificity of the intact antibody from which it is derived. The light and heavy chains may be in any order, for example, VH-linker-VL or VL-linker-VH, as long as the specificity of the scFv for the target antigen is retained. In the specification, the linker may be exemplified by a flexible linker peptide chain rich in glycine and serine.

The term "chimeric antigen receptor" (CAR) in the specification refers to an artificially engineered receptor that can anchor a specific molecule that recognizes a tumor cell surface antigen (e.g., an antibody) to immune cells (e.g., T cells) to enable the immune cells to recognize the tumor antigen or a viral antigen and kill tumor cells or virus-infected cells. A CAR typically comprises an optional signal peptide, a polypeptide that binds to a tumor cell membrane antigen (e.g., a single-chain antibody), a hinge region, a transmembrane region, and an immunocompetent cell activation signal transduction region in sequence, wherein the immunocompetent cell activation signal transduction region comprises a co-stimulatory domain and a signal transduction domain.

"Hinge region" refers to a hydrophilic region between an antigen recognition domain and a transmembrane domain. The hinge region may be a hinge region of various different antibodies or antigen receptors, particularly the hinge region of a CD molecule. In one specific embodiment, the hinge region may be selected from, for example, CD4, CD8α, CD28, IgG1, and IgG4. In one preferred embodiment of the present disclosure, the CD8α hinge region is used. Examples of sequences useful as hinge regions include:

CD8 hinge region (SEQ ID NO: 21):

TTCGTGCCGGTCTTCCTGCCAGCGAAGCCCACCACGACGCCAGCGCCGCG

ACCACCAACACCGGCGCCCACCATCGCGTCGCAGCCCCTGTCCCTGCGCC

CAGAGGCGTGCCGGCCAGCGGCGGGGGGCGCAGTGCACACGAGGGGGCT

GGACTTCGCCTGTGAT

The "transmembrane region" only needs to include a peptide capable of penetrating through the cell membrane. A preferably used transmembrane region is the transmembrane region of a CD molecule. In one embodiment, the transmembrane region may be selected from, for example, the transmembrane regions of CD4, CD8, CD28, CD3ζ, the α and β chains of T cell receptors, CD3ε, CD45, CD5, CD9, CD16, CD22, CD33, CD37, CD64, CD80, CD86, CD134, CD137, ICOS, CD154, EGFR (epidermal growth factor receptor), NKG2D, and GITR. In one preferred embodiment of the present disclosure, the CD8α transmembrane region is used. Examples of sequences useful as transmembrane regions include:

CD8 transmembrane region (SEQ ID NO: 22):

ATCTACATCTGGGCGCCCTTGGCCGGGACTTGTGGGGTCCTTCTCCTGTCA

CTGGTTATCACCCTTTACTGCAACCACAGGAAC

CD4 transmembrane region (SEQ ID NO: 23):

ATGGCCCTGATTGTGCTGGGGGGCGTCGCCGGCCTCCTGCTTTTCATTGGG

CTAGGCATCTTCTTC

The term "co-stimulatory domain" refers to the portion of a CAR that enhances the proliferation, survival, and/or development of memory cells. The CAR of the present disclosure may comprise one or more co-stimulatory domains. Each of the co-stimulatory domains comprises, for example, a co-stimulatory domain of any one or more of: CD28, 4-1BB, ICOS, OX40, CD27, CD40, Myd88, HVEM, GITR, Fc receptor-associated γ chain, and the like. In one preferred embodiment of the present disclosure, a combination of CD28 and 4-1BB is used. Examples of sequences useful as co-stimulatory domains include:

4-1BB (SEQ ID NO: 24):

CGTTTCTCTGTTGTTAAACGGGGCAGAAAGAAGCTCCTGTATATATTCAA

ACAACCATTTATGAGACCAGTACAAACTACTCAAGAGGAAGATGGCTGT

AGCTGCCGATTTCCAGAAGAAGAAGAAGGAGGATGTGAACTG

CD28 (SEQ ID NO: 25):

AGGAGTAAGAGGAGCAGGCTCCTGCACAGTGACTACATGAACATGACTC

CCCGCCGCCCCGGGCCCACCCGCAAGCATTACCAGCCCTATGCCCCACCA

CGCGACTTCGCAGCCTATCGCTCC

The term "signal transduction domain" refers to the portion of a CAR that transduces effector function signals and directs cells to perform their specialized functions. Examples of domains that transduce effector function signals include, but are not limited to, CD3ζ and FcεRIγ. In one preferred embodiment of the present disclosure, the CD3ζ domain is used. Examples of sequences useful as signal transduction domains include:

CD3z (SEQ ID NO: 26):

AGAGTGAAGTTCAGCAGGAGCGCAGACGCCCCCGCGTACCAGCAGGGCC

AGAACCAGCTCTATAACGAGCTCAATCTAGGACGAAGAGAGGAGTACGA

TGTTTTGGACAAGAGACGTGGCCGGGACCCTGAGATGGGGGGGAAAGCCG

AGAAGGAAGAACCCTCAGGAAGGCCTGTACAATGAACTGCAGAAAGATA

AGATGGCGGAGGCCTACAGTGAGATTGGGATGAAAGGCGAGCGCCGGAG

GGGCAAGGGGCACGATGGCCTTTACCAGGGTCTCAGTACAGCCACCAAG

GACACCTACGACGCCCTTCACATGCAGGCCCTGCCCCCTCGC

In the phrase "a membrane protein, a secreted protein, an intracellular protein, a small-molecule drug, and a cytotoxic drug" of the specification, the membrane protein refers to a protein that is entirely or partially inserted into various membrane structures (including membrane structures such as the cell membrane, the mitochondrial membrane, the endoplasmic reticulum membrane, the nuclear membrane, and the like); the secreted protein refers to a protein that is secreted to the outside of a cell after being synthesized; the intracellular protein refers to all proteins present within the cell membrane, including free proteins that are free in the cytoplasm and membrane proteins on the membranes of various intracellular organelles; the small-molecule drug refers to a chemically synthesized drug with a molecular weight of less than 1000 that can be linked to another protein or molecular drug by a linking arm; the cytotoxic drug refers to a drug that has a toxic effect on cells or particular cells, including but not limited to alkaloid drugs (e.g., paclitaxel), metabolic drugs (e.g., decitabine), antibiotic drugs (e.g., idarubicin), alkylating drugs (e.g., ifosfamide), platinum-based agents (e.g., cisplatin) and the like, which can also be linked to another protein or molecular drug by a linking arm.

In the chimeric antigen receptor of the present disclosure, examples of membrane proteins include CD40L, CXCR5, CXCR3, 4-1BB, ICOS, OX40, CD27, NKG2D, and the like; examples of secreted proteins include IL2, IL15, IL4, IL7, IL10, IL18, IFNγ, IL1β, and antibodies (e.g., anti-PD1 antibodies, anti-CTLA4 antibodies, and the like); examples of small-molecule drugs include FITC/folic acid, rapamycin, rimiducid, PROTAC compounds, dasatinib, and the like; and examples of cytotoxic drugs include paclitaxel, vinorelbine, docetaxel, hydroxycamptothecin, gemcitabine, cytarabine, tegafur, methotrexate, epirubicin, pirarubicin, idarubicin, mitomycin, mitoxantrone, ifosfamide, dacarbazine, cisplatin, oxaliplatin, and the like.

The term "nucleic acid molecule" in the specification refers to a biomolecular compound formed by polymerizing deoxyribonucleic acid (DNA) or ribonucleic acid (RNA). The components of a nucleic acid molecule are nucleotides, and a nucleotide monomer consists of a pentose sugar, a phosphate, and a nitrogenous base.

When applied to a nucleic acid sequence, the term "encode" in the specification means that a polynucleotide "encoding" a polypeptide, in its natural state or when manipulated by methods well known to those skilled in the art, can be transcribed and/or translated to produce an mRNA for the polypeptide and/or a fragment thereof.

The term "vector" in the specification refers to a recombinant vector that retains the ability to infect and transduce non-dividing and/or slowly dividing cells and integrate into the genome of the target cell. In some preferred embodiments, the vector is derived from or based upon a wild-type virus. In some other further preferred embodiments, the vector is derived from or based upon a wild-type lentivirus. Examples of the vector include retroviral vectors, lentiviral vectors, adenoviral vectors, adeno-associated virus vectors, herpes simplex virus vectors, and the like. More specifically, in one preferred embodiment of the present disclosure, the vector is a lentiviral vector; and in another preferred embodiment of the present disclosure, the vector is a retroviral vector.

The term "autologous or allogeneic" in the specification refers to "autologous cells" or "allogeneic cells". "Autologous cells" refer to cells derived from the same individual that are subsequently re-administered to the individual; "allogeneic cells" refer to cells other than autologously derived cells.

Examples of the cell of the present disclosure include T cells, B cells, NK cells, macrophages, monocytes, dendritic cells, neutrophils, basophils, eosinophils, mast cells, NK-T cells, MAIT cells, hematopoietic stem cells, embryonic stem cells, induced pluripotent stem cells, and erythrocytes, wherein examples of the T cells include αβ T cells, γδ T cells, and regulatory T cells. In one preferred embodiment of the present disclosure, γδ T cells are used.

The pharmaceutical composition of the present disclosure comprises any one of the antibody, CAR, nucleic acid molecule, vector, and cell of the present disclosure, and may further comprise a pharmaceutically acceptable carrier. In addition, the pharmaceutical composition of the present disclosure may comprise any two or more of the antibody, CAR, nucleic acid molecule, vector, and cell of the present disclosure, e.g., the CAR and the nucleic acid molecule, and may further comprise a pharmaceutically acceptable carrier. In addition, the pharmaceutical composition of the present disclosure may comprise another pharmaceutically active agent or drug, such as a chemotherapeutic agent, for example, asparaginase, busulfan, carboplatin, cisplatin, daunorubicin, doxorubicin, fluorouracil, gemcitabine, hydroxyurea, methotrexate, paclitaxel, rituximab, vinblastine, vincristine, or the like. In one preferred embodiment, the pharmaceutical composition comprises the cell of the present disclosure.

In addition, the pharmaceutically acceptable carrier in the pharmaceutical composition may be any conventionally used pharmaceutically acceptable carrier, and those skilled in the art can select a suitable pharmaceutically acceptable carrier according to conditions such as chemical-physical conditions, the route of administration, and the like. Examples of the pharmaceutically acceptable carrier described in the specification include vehicles, adjuvants, excipients, diluents, and the like. The pharmaceutically acceptable carrier is preferably a carrier that is chemically inert to the active agent and a carrier that does not have deleterious side effects or toxicity under use conditions. The term "treatment or prevention" in the specification refers to, after a certain disease occurs, a way capable of eliminating the disease by a certain means or a way of avoiding the disease by a certain means. The terms "treatment" and "prevention" in the specification do not necessarily mean 100% or complete treatment or prevention. Rather, there are varying degrees of treatment or prevention that those skilled in the art would recognize as having potential beneficial or therapeutic effects. In addition, the treatment or prevention provided by the method of the present disclosure may include treating or preventing one or more conditions or symptoms of the disease being treated or prevented (e.g., cancer). Moreover, when used for the purposes of the present disclosure, "prevention" may include delaying the onset of the disease or a symptom or condition thereof.

The term "subject" in the specification means a mammal. In general, the subject of the present disclosure refers to any subject, preferably a human, afflicted with cancer or at risk of being afflicted with cancer.

The term "therapeutically effective amount" in the specification refers to an amount of the antibody or antibody fragment, chimeric antigen receptor, nucleic acid molecule, vector, cell, or pharmaceutical composition of the present disclosure that is sufficient to treat B7-H3-positive diseases (for example, limit growth or slow or block tumor metastasis) at a reasonable benefit/risk ratio applicable to any medical treatment. However, it should be appreciated that the total daily usage of the antibody or antibody fragment, chimeric antigen receptor, nucleic acid molecule, vector, cell, or pharmaceutical composition of the present disclosure will be decided by the attending physician within the scope of sound medical judgment. The specific therapeutically effective dose level for any particular subject will depend upon a variety of factors, including the condition being treated and the severity of the condition, the activity of the specific substance administered, the specific combination used, the age, body weight, general health, gender, and diet of the subject, the time of administration, route of administration, and rate of excretion of the specific substance administered, the duration of the treatment, the drugs used in combination or concurrently with the specific substance administered, and similar factors well known in the medical field.

The term "B7-H3-positive disease" in the specification refers to a disease associated with high expression of B7-H3, including malignant/benign hematological tumors, malignant/benign solid tumors, autoimmune diseases, bacterial infections, viral infections, parasitic infections, abnormal bone growth, allogeneic transplantation, transplant rejection, and the like, including acute myeloid leukemia, chronic myeloid leukemia, acute lymphocytic leukemia, non-Hodgkin lymphoma, multiple myeloma, melanoma, lung cancer, colorectal cancer, renal tumors, bladder cancer, gastrointestinal cancer, prostate cancer, liver cancer, ovarian cancer, pancreatic cancer, endometrial cancer, gastric cancer, prostate cancer, kidney cancer, cervical cancer, thyroid cancer, uterine cancer, neuroendocrine cancer, head and neck cancer, nasopharyngeal carcinoma, testicular cancer, basal cell skin cancer, squamous cell skin cancer, dermatofibrosarcoma protuberans, Merkel cell carcinoma, glioblastoma, glioma, sarcomas, mesothelioma, myelodysplastic syndrome, or the like, wherein the autoimmune diseases include systemic lupus erythematosus, rheumatoid arthritis, multiple sclerosis, Sjogren's syndrome, ankylosing spondylitis, and the like. The kit for detection of the present disclosure is a kit for detecting B7-H3, and the kit usually comprises components usually used in such kits, for example, a pH buffer, a stabilizer, and attached documents such as a manual, instructions for detecting B7-H3, and the like.

The isolation kit of the present disclosure is an isolation kit for isolating B7-H3-positive cells, and the kit usually comprises components usually used in such kits, for example, a pH buffer, a stabilizer, and attached documents such as a manual, instructions for isolating B7-H3-positive cells, and the like.

In addition, the term "P2A cleavage peptide" in the specification refers to a virus-derived short peptide (18 to 25 amino acids in length) that, through a peptide called "self-cleavage", enables one transcript to produce a variety of proteins. The fundamental mechanism for this is to act by making the ribosome skip the synthesis of the peptide bond between glycine and proline at the C-terminus of the 2A element, resulting in the separation of the 2A sequence terminus from the downstream product. The P2A polypeptide is one of the polypeptides, and the other polypeptides include T2A, E2A, and F2A. The four 2A peptides are derived from different viruses.

The term "truncated EGFR" in the specification, abbreviated as "EGFRt", refers to a truncated form of wild-type EGFR with domain III and domain IV kept. This truncated EGFR has no intracellular signal and does not transmit other signals into T cells. Moreover, the two domains are epitopes recognized by cetuximab. When expressed on T cells, the epitopes can not only serve as a screening label for CAR-T but also provide a safety switch for clinical studies. EGFRt can be specifically bound through an inducer (e.g., cetuximab), and with the help of antibody-dependent cell-mediated cytotoxicity, the apoptosis of CAR-T cells is achieved, so that CAR-T cells in the body can be eliminated at any time.

The term "antibody-dependent cell-mediated cytotoxicity" described above, abbreviated as ADCC, means that the Fab fragment of an antibody binds to an antigenic epitope of a tumor cell, and its Fc fragment binds to the FcR on the surface of a killer cell (NK cell, macrophage, etc.), mediating the direct killing of the target cell by the killer cell.

The term "flexible linker peptide chain" in the specification refers to an oligopeptide or polypeptide region that is about 1 to 100 amino acids in length and links any domain/region of the CAR of the present disclosure. A linker may consist of flexible residues (e.g., glycine and serine), so that adjacent protein domains are free to move relative to each other. When it is desired to ensure that two adjacent domains do not sterically interfere with each other, a longer linker can be used. In one preferred embodiment of the present disclosure, $(GGGGS)_3$ is used.

The term "transduction positivity rate" in the specification refers to the proportion of transduction of an exogenous gene on T cells, including the rate of transduction of the CAR on T cells, which represents the proportion of CAR-positive T cells to total T cells.

In the specification, "affinity" or "binding affinity" or "KD" is determined by measuring the equilibrium association constant (ka) and the equilibrium dissociation constant (kd) and calculating the quotient of kd and ka (KD = kd/ka). A smaller KD value indicates a stronger affinity, and a larger KD value indicates a weaker affinity.

Experimental Materials and Methods

1. Culture of tumor cell lines

Six adherent tumor cell types are used in this study, and they are 293T, U87, HTB15, TJ905, RKO, and SKOV3 cells. 293T cells are a human kidney epithelial cell line that is often used for studying the expression of exogenous genes, virus preparation, and the like. U87, HTB15, and TJ905 are all human brain glioma cell lines. RKO is a human colon cancer cell line. SKOV3 is a human ovarian cancer cell line. The tumor cells other than SKOV3 are cultured in DMEM complete culture medium (DMEM culture medium + 10% FBS + 1% double antibody). SKOV3 is cultured in McCoy's 5a complete culture medium (McCoy's 5a culture medium + 20% FBS + 1% double antibody).

Jurkat T cells are a human T lymphocyte leukemia cell line, and the cells are cultured in RPMI-1640 complete culture medium (RPMI-1640 culture medium + 10% FBS + 1% double antibody).

2. Lentivirus production

$1 \times 10^7$ 293T cells are seeded into a polylysine-coated 10-cm cell culture dish, with a culture medium volume of 10 mL. The following day, the expression plasmid (the backbone is from pCDH-EF1-MCS-T2A-copGFP plasmid (Addgene, Plasmid #72263)), the helper plasmid pspAx2 (Addgene, #12260), and the helper plasmid pCMV-VSV-G (Addgene, Plasmid #8454) are homogeneously mixed in amounts of 6 $\mu$g, 4 $\mu$g, and 2 $\mu$g in 300 $\mu$L of opti-MEM culture medium to prepare a plasmid-containing culture medium. In addition, after 30 $\mu$g of PEI is added to 300 $\mu$L of opti-MEM culture medium and mixed thoroughly and homogeneously, the PEI solution is added to the plasmid-containing culture medium described above and immediately homogeneously mixed, and the mixture is left to stand at room temperature for 15 min and homogeneously added to the 293T cell culture dish (this moment is 0 h). After 8 h, the culture medium is replaced with a new 10 mL of DMEM complete culture medium. Culture medium supernatants are collected at 48 h and 72 h, respectively, with another 8 mL of fresh DMEM complete culture medium added after the supernatant collection at 48 h. The supernatants are concentrated using a Lenti-X™ Concentrator (Takara) to obtain virus liquids, and the viral titers of the virus liquids are measured using 293T cells. The virus liquids are aliquoted and subjected to long-term storage at -80 °C.

3. Culture of primary cells

(1) $\gamma\delta$ T cells

PBMCs are prepared and washed twice with PBS. The cells are counted using an AO/PI cell counter, and the viability is measured.

The PBMCs are cultured in an RPMI1640 culture medium + 10% FBS + 1% double antibody + 200 U/mL rhIL-2 culture system, with an initial cell density of $2 \times 10^6$ cells/mL. At the same time, 5 $\mu$M zoledronic acid (ZOL) is added. After 48 h, an equal volume liquid supplementation (no more ZOL is added) is performed. Subsequently, the cell density ($1 \times 10^6$ cells/mL) is adjusted once every 48 h.

(2) $\alpha\beta$ T cells

PBMCs are prepared and washed twice with PBS. The cells are counted using an AO/PI cell counter, and the viability is measured.

Coating of 6-well plates with antibodies: CD3 and CD28 antibodies are diluted with opti-MEM culture medium to 200 ng/mL, 2 mL of antibody dilution is added to each well of 6-well plates, and the plates are incubated overnight at 4 °C or incubated at 37 °C for 2 h.

Immediately before PBMCs are added to the 6-well plates, the antibody dilutions are pipetted off, and the plates are washed once with 2 mL of PBS.

Subsequently, PBMCs are added. The PBMCs are resuspended in an RPMI1640 culture medium + 10% FBS + 1% double antibody + 200 U/mL rhIL-2 culture system, and the cell density is adjusted to $2 \times 10^6$ cells/mL. Subsequently, the cell density is adjusted (to $1 \times 10^6$ cells/mL) with the RPMI1640 culture medium + 10% FBS + 1% double antibody + 200 U/mL rhIL-2 culture system once every 48 h.

### 4. Viral transduction of $\alpha\beta$ T and $\gamma\delta$ T

A $1 \times 10^7$ TU lentivirus liquid is diluted in 200 $\mu$L of PBS, and the dilution is added to a 24-well plate. The 24-well plate is coated with RetroNectin in advance. The plate is centrifuged at 2000 g at 32 °C for 2 h. After the centrifugation, the virus dilution is pipetted off, and the plate is washed three times in total with 1 mL of PBS. Then cells are added.

Regarding the transduction of $\alpha\beta$ T or $\gamma\delta$ T cells: $\alpha\beta$ T or $\gamma\delta$ T cells after 24 h of *in vitro* activation culture are counted. After $1 \times 10^6$ total cells are resuspended in a 1 mL RPMI1640 culture medium + 10% FBS + 1% double antibody + 200 U/mL rhIL-2 culture system, the suspension is added to the virus-treated 24-well plate. The plate is centrifuged at 800 g at 32 °C for 10 min and then incubated in a cell incubator. Subsequently, cell counting is performed once every two to three days, with the density adjusted to $1 \times 10^6$ cells/mL.

### 5. Flow cytometry staining analysis

Cells to be analyzed by flow cytometry are washed once with PBS. $2 \times 10^5$ cells are stained with a 50 $\mu$L staining system. After the cells are resuspended in the staining system, the suspension is incubated in a 4 °C environment for 30 min. After the incubation, the cells are washed twice with flow cytometry buffer, resuspended in 200 $\mu$L of flow cytometry buffer, and then analyzed on a flow cytometer.

The staining system is: 50 $\mu$L of flow cytometry buffer + antibody.

The formula for the flow cytometry buffer is: PBS + 1% FBS + 2.5 mM EDTA.

The amount of an antibody used depends upon the concentration of the antibody and the actual situation. For most antibodies (at a concentration of 0.5-1 mg/mL), a dilution ratio of 1:50-1:100 can meet the requirements.

The antibodies used in this assay mainly include: anti-human CD3, anti-human $\alpha\beta$ TCR, anti-human $\gamma\delta$ TCR, and anti-human EGFR.

### 6. CD69 expression assay

Effector cells and tumor cells are incubated in an effector-to-target ratio of 1:1 in a 24-well plate, with $0.5 \times 10^6$ effector cells and $0.5 \times 10^6$ tumor cells added. After 24 h of incubation, flow cytometry analysis is performed.

For Jurkat T cells, the flow cytometry staining system is: anti-human EGFR and anti-human CD69. For $\alpha\beta$ T cells, the flow cytometry staining system is: anti-human CD3, anti-human $\alpha\beta$ TCR, anti-human EGFR, and anti-human CD69.

### 7. *In vitro* cell killing assay

The *in vitro* cell killing assay is based on the luciferase fluorescence detection method. The tumor cells to be tested stably express luciferase. Killing tests are performed in a 24-well plate, with different effector-to-target ratios set according to experimental requirements. If the effector-to-target ratio is 1:1, tumor cells and effector cells are counted separately, and the two types of cells are resuspended to $0.5 \times 10^6$ cells/mL in their respective culture media. 500 $\mu$L of tumor cells and 500 $\mu$L of effector cells are added to the same well and mixed thoroughly and homogeneously. A control well containing only tumor cells is set, with 500 $\mu$L of tumor cell culture medium and 500 $\mu$L of effector cell culture medium added. The plate is incubated in a cell incubator. After 24 h, luciferase fluorescence signals are detected.

$$\text{Killing efficiency (cytotoxicity\%)} = \frac{\text{Control well signal value} - \text{Test well signal value}}{\text{Control well signal value}} \times 100\%$$

8. B7-H3 and CAR-B7-H3 specific binding assay

The B7-H3 and CAR-B7-H3 specific binding is assessed by flow cytometry. Specifically, CAR-B7-H3 is stably expressed on Jurkat T cells. In a 100 μL flow cytometry buffer staining system, biotin-B7-H3 antigen, at different concentrations (ranging from 0.03125 μg/mL to 16 μg/mL), is incubated with $1 \times 10^5$ CAR-B7-H3-Jurkat T cells at 4 °C for 45 min. After the incubation, the mixture is centrifuged at 500 g for 5 min, and the supernatant is discarded. After the cells are resuspended in 500 μL of flow cytometry buffer, the suspension is centrifuged at 500 g for 5 min, and the supernatant is discarded. The cells are resuspended in 100 μL of flow cytometry buffer, and 0.5 μL of fluorescently labeled streptavidin secondary antibody and 0.5 μL of fluorescently labeled anti-human EGFR antibody are added simultaneously. The mixture is incubated at 4 °C for 30 min. After the incubation, the mixture is centrifuged at 500 g for 5 min, and the supernatant is discarded. After the cells are resuspended in 300 μL of flow cytometry buffer, the fluorescence signals of the streptavidin secondary antibody in the EGFR-positive population are analyzed by flow cytometry.

9. Antibody affinity assay

B7-H3 antigen protein solutions are prepared, with 7 concentrations of serial dilution of 100 nM, 50 nM, 25 nM, 12.5 nM, 6.25 nM, 3.13 nM, and 1.56 nM. The samples are loaded onto a 96-well plate. A human Protein G probe is selected. The stationary phase is the antigen, and the mobile phase is a B7-H3 antibody. The association time is set to 180 s, and the dissociation time is set to 300 s. The association and dissociation constants of the human monoclonal antibody are determined, and the affinity is calculated.

10. Assessment of the Antigen-specific binding of B7-H3 antibody by ELISA

Antigens to be tested, including B7-H1, B7-H3, B7-H4, and B7-H5, are each serially diluted with PBS to 5 concentrations of 0.5 mg/mL, 1 mg/mL, 1.5 mg/mL, 2 mg/mL, and 4 mg/mL and plated onto a microplate (96-well plate), with 100 μL added to each well. The plate is left to stand at room temperature for 1 h. The plate is washed twice with PBST, with 100 μL per well; after addition, the plate is left to stand for 5 min. Subsequently, a B7-H3 antibody diluted with PBS to 0 μg/mL, 2 μg/mL, or 4 μg/mL is added at 100 μL per well. The plate is left to stand at room temperature for 1 h. The plate is washed three times with PBST, with 100 μL per well; after addition, the plate is left to stand for 5 min. The HRP-anti-human Fc antibody is diluted with skimmed milk in a 1:2000 ratio (the antibody concentration is 0.5 mg/mL) and added at 100 μL per well, and the plate is left to stand at room temperature for 1 h. The plate is washed three times with PBST, with 100 μL per well; after addition, the plate is left to stand for 5 min. The TMB substrate solution is added at 100 μL per well in a dark place, and the plate is left to stand in a dark place at room temperature for 40 min. 100 μL of 2 M sulfuric acid is added to each well to terminate the reaction. The microplate is placed on a microplate reader, and the OD values at 450 nm are measured.

11. Parent antibody blocking assay

100 μL of B7-H3 protein, diluted with PBS to 10 μg/mL, is added to a 96-well microplate and incubated overnight at 4 °C. The plate is washed twice with PBST, with 100 μL per well; after addition, the plate is left to stand for 5 min. 100 μL of a murine parent antibody diluted with PBS to 0.625, 1.25, 2.5, 5, 10, 20, and 40 μg/mL is added and reacted for 1 h. The plate is washed twice with PBST, with 100 μL per well; after addition, the plate is left to stand for 5 min. Then 100 μL of a B7-H3 antibody diluted with PBS to 2 μg/mL and 100 μL of antibody 8H9 (which recognizes a different antigenic epitope of B7-H3), diluted with PBS to 2 μg/mL, are added. After incubation, analysis is performed by ELISA (see item 10 of Experimental Materials and Methods).

Examples

The technical solutions provided in the present disclosure are further described using the specific examples below, which are intended to be illustrative only of the present disclosure rather than limit the scope of the present disclosure.

In addition, the various experimental methods in the examples are all conventional methods unless otherwise specified. The various reagent materials and the like used in the examples are all commercially available products unless otherwise specified.

Example 1: Assay for Affinity of Humanized 1B4 Antibody

The objective of this example is to assess the affinity of a humanized 1B4 antibody for the antigen B7-H3. B7-H3 antigen protein solutions were prepared, with 7 concentrations of serial dilution of 100 nM, 50 nM, 25 nM, 12.5 nM, 6.25 nM, 3.13 nM, and 1.56 nM. The samples were loaded onto a 96-well plate. A human Protein G probe was selected. The stationary phase was the antigen, and the mobile phase was 1B4. The association time was set to 180 s, and the dissociation time was set to 300 s. The association and dissociation constants of the human monoclonal antibody were determined, and the affinity was calculated.

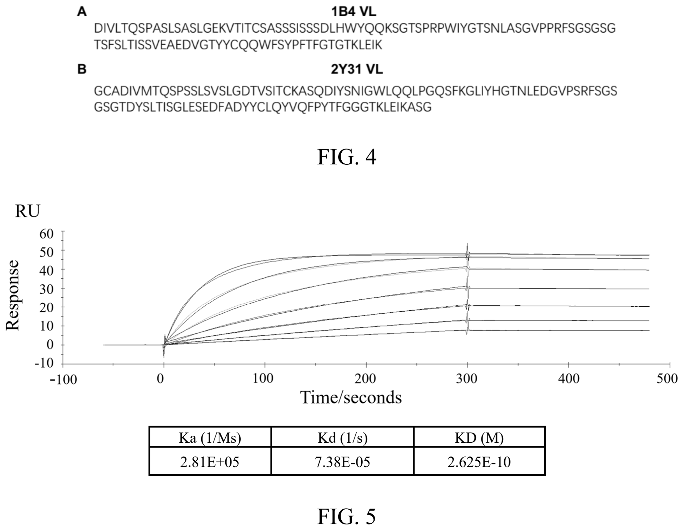

The results are shown in FIG. 5. The results show that the association constant ka was 2.81E+05, the dissociation constant kd was 7.38E-05, and the equilibrium dissociation constant KD was 2.625E-10.

Example 2: Parent Antibody Blocking Assay

100 μL of B7-H3 protein, diluted with PBS to 10 μg/mL, was added to a 96-well microplate and incubated overnight at 4 °C. The plate was washed twice with PBST, with 100 μL per well; after addition, the plate was left to stand for 5 min. 100 μL of a murine parent B7-H3 antibody (m1B4 or m2Y31) diluted with PBS to 0.625, 1.25, 2.5, 5, 10, 20, and 40 μg/mL was added and reacted for 1 h. The plate was washed twice with PBST, with 100 μL per well; after addition, the plate was left to stand for 5 min. Then 100 μL of a humanized B7-H3 antibody (hu1B4 or hu2Y31) diluted with PBS to 2 μg/mL or 100 μL of antibody 8H9 (which recognizes a different antigenic epitope of B7-H3), diluted with PBS to 2 μg/mL, was added. After incubation, analysis was performed by ELISA.

The results are shown in Table 1.

Table 1. The results of the parent antibody blocking assay

| | | OD value (450 nm) | | | |
|---|---|---|---|---|---|
| | | hu1B4 | | 8H9 | |
| m1B4 | 40 μg/mL | 0.965 | 0.955 | 3.234 | 3.248 |
| | 20 μg/mL | 1.022 | 1.028 | 3.209 | 3.273 |
| | 10 μg/mL | 1.135 | 1.148 | 3.288 | 3.244 |
| | 5 μg/mL | 1.201 | 1.183 | 3.122 | 3.179 |
| | 2.5 μg/mL | 1.334 | 1.334 | 3.224 | 3.204 |
| | 1.25 μg/mL | 1.708 | 1.716 | 3.211 | 3.163 |
| | 0.625 μg/mL | 2.409 | 2.422 | 3.302 | 3.287 |
| | 0 μg/mL | 3.231 | 3.185 | 3.376 | 3.391 |
| | | OD value (450 nm) | | | |
| | | hu2Y31 | | 8H9 | |
| m2Y31 | 40 μg/mL | 0.854 | 0.937 | 2.403 | 2.428 |
| | 20 μg/mL | 0.745 | 0.72 | 2.428 | 2.439 |
| | 10 μg/mL | 0.703 | 0.684 | 2.403 | 2.444 |
| | 5 μg/mL | 0.65 | 0.651 | 2.459 | 2.448 |
| | 2.5 μg/mL | 0.645 | 0.606 | 2.391 | 2.41 |
| | 1.25 μg/mL | 0.636 | 0.644 | 2.28 | 2.374 |
| | 0.625 μg/mL | 0.626 | 0.632 | 2.375 | 2.392 |
| | 0 μg/mL | 2.069 | 2.02 | 2.358 | 2.359 |

The assay shows that the addition of the 1B4 or 2Y31 parent antibody significantly reduced the ability of the corresponding humanized 1B4 or 2Y31 antibody to bind to B7-H3 antigen and did not significantly affect antibody 8H9, which recognizes a different epitope. It can be seen from the results that m1B4 at a mere concentration of 0.625 µg/mL blocked the binding of hu1B4 to antigen molecules, suggesting that hu1B4 and m1B4 recognize the same site. Neither low nor high concentrations of m1B4 blocked the binding of 8H9 to antigen molecules, further verifying that 1B4 and 8H9 recognize different sites. Likewise, m2Y31 at a mere concentration of 0.625 µg/mL blocked the binding of hu2Y31 to antigen molecules, suggesting that hu2Y31 and m2Y31 recognize the same site, which differs from the site recognized by 8H9.

Example 3: Determination of Expression Levels of B7-H3 in Wild-Type U87 Tumor Cells and B7-H3 Knockout U87 Tumor Cells (U87-B7-H3 CAS9)

A B7-H3 knockout U87 tumor cell strain was constructed using CRISPR-Cas9 technology. A million human glioma U87 cells were washed once with PBS and then resuspended in 100 µL of PBS. Subsequently, 1 µg of a corresponding reagent was added to each group (the negative control: NC; the positive control: the B7-H3 antibody 8H9; the experimental groups: the humanized 1B4 antibody, the humanized 2Y31 antibody, the parent murine 1B4 antibody, and the parent murine 2Y31 antibody). The cells were incubated at 4 °C for 30 min, washed once with PBS, resuspended in 500 µL of PBS, and analyzed on an instrument for their ability to bind to B7-H3 antigen.
The same procedure was performed except that a million B7-H3 knockout U87 cells were used in place of the million human glioma U87 cells in the procedure described above.
The results are shown in FIG. 6. As shown in FIG. 6A, the signal (dashed-line peak) of the humanized 1B4 antibody binding to the glioma cell strain, the U87 cell strain, was significantly higher than that of the parent murine 1B4 antibody (solid-line peak). Moreover, after the knockout of the B7-H3 of the U87 cell strain, neither antibody exhibited a binding effect. These indicate the specific binding of antibody 1B4 to B7-H3 antigen.
The results in FIG. 6B show that the signal of the humanized 2Y31 antibody (hu2Y31) binding to the U87 cell strain was significantly higher than that of the parent murine 2Y31 antibody (m2Y31) and than that of antibody 8H9. All antibodies exhibited no binding signal to B7-H3 knockout U87 tumor cells. These indicate that antibody 2Y31 is specific to B7-H3 antigen, and that the ability of the humanized antibody to bind to B7-H3 antigen is higher than that of the parent murine antibody.

Example 4: Assay for Ability of Antibody 1B4 to Bind to Other Proteins in Same Family as B7-H3

To verify the specificity of the binding of antibody 1B4 to B7-H3 protein to exclude its binding to other members of the B7 family, tests were performed by ELISA in this patent. Antigens to be tested, including B7-H1, B7-H3, B7-H4, and B7-H5, were each serially diluted with PBS to 5 concentrations of 0.5 mg/mL, 1 mg/mL, 1.5 mg/mL, 2 mg/mL, and 2.5 mg/mL and plated onto a microplate (96-well plate), with 100 µL added to each well. The plate was left to stand at room temperature for 1 h. The plate was washed twice with PBST, with 100 µL per well; after addition, the plate was left to stand for 5 min. Subsequently, the B7-H3 antibody, diluted with PBS to 0 µg/mL, 2 µg/mL, or 4 µg/mL, was added at 100 µL per well. The plate was left to stand at room temperature for 1 h. The plate was washed three times with PBST, with 100 µL per well; after addition, the plate was left to stand for 5 min. The HRP-anti-human Fc antibody was diluted with skimmed milk in a 1:2000 ratio (the antibody concentration was 0.5 mg/mL) and added at 100 µL per well, and the plate was left to stand at room temperature for 1 h. The plate was washed three times with PBST, with 100 µL per well; after addition, the plate was left to stand for 5 min. The TMB substrate solution was added at 100 µL per well in a dark place, and the plate was left to stand in a dark place at room temperature for 40 min. 100 µL of 2 M sulfuric acid was added to each well to terminate the reaction. The microplate was placed on a microplate reader, and the OD values at 450 nm were measured. The results are shown in Table 2.

Table 2. The results of the assay for the ability of antibody 1B4 to bind to other proteins in the same family as B7-H3 (OD450)

| | B7-H1 | | | B7-H3 | | | B7-H4 | | | B7-H5 | |
|---|---|---|---|---|---|---|---|---|---|---|---|
| µg/mL | c=0.5 mg/mL | c=2 mg/mL | µg/mL | c=0.5 mg/mL | c=2 mg/mL | µg/mL | c=0.5 mg/mL | c=2 mg/mL | µg/mL | c=0.5 mg/mL | c=2 mg/mL |
| 4 | 0.0506 | 0.0493 | 4 | 3.6722 | 3.6537 | 4 | 0.0471 | 0.0507 | 4 | 0.0494 | 0.0501 |
| 2 | 0.0472 | 0.0472 | 2 | 3.6925 | 3.6532 | 2 | 0.0491 | 0.0507 | 2 | 0.0500 | 0.0472 |
| 0 | 0.0460 | 0.0445 | 0 | 0.0410 | 0.0478 | 0 | 0.0486 | 0.0492 | 0 | 0.0476 | 0.0483 |
| | c=1mg/mL | c=4 mg/mL | | c=1 mg/mL | c=4 mg/mL | | c=1 mg/mL | c=4 mg/mL | | c=1 mg/mL | c=4 mg/mL |

(continued)

| | B7-H1 | | | B7-H3 | | | B7-H4 | | | B7-H5 | |
|---|---|---|---|---|---|---|---|---|---|---|---|
| µg/mL | c=0.5 mg/mL | c=2 mg/mL | µg/mL | c=0.5 mg/mL | c=2 mg/mL | µg/mL | c=0.5 mg/mL | c=2 mg/mL | µg/mL | c=0.5 mg/mL | c=2 mg/mL |
| 4 | 0.0467 | 0.0490 | 4 | 3.6864 | 3.7618 | 4 | 0.0509 | 0.0481 | 4 | 0.0467 | 0.0481 |
| 2 | 0.0476 | 0.0487 | 2 | 3.7190 | 3.8095 | 2 | 0.0502 | 0.0477 | 2 | 0.0450 | 0.0431 |
| 0 | 0.0457 | 0.0400 | 0 | 0.0455 | 0.0467 | 0 | 0.0488 | 0.0485 | 0 | 0.0480 | 0.0451 |
| | c=1.5 mg/mL | | | c=1.5 mg/mL | | | c=1.5 mg/mL | | | c=1.5 mg/mL | |
| 4 | 0.0414 | | 4 | 3.7183 | | 4 | 0.0491 | | 4 | 0.0499 | |
| 2 | 0.0462 | | 2 | 3.7589 | | 2 | 0.0500 | | 2 | 0.0481 | |

As shown in Table 2, antibody 1B4 at a mere concentration of 2 µg/mL exhibited a very high ability to bind to B7-H3 antigen at 0.5 mg/mL, with its OD450 value exceeding 3.6 and reaching a saturation value; further increasing the concentration of antibody 1B4 or B7-H3 antigen did not result in a higher OD450 value. By contrast, 1B4 did not exhibit an ability to bind to B7-H1, B7-H4, or B7-H5. Increasing the concentration of antibody 1B4 or B7-H3 antigen did not increase the OD450 values, which all remained at the baseline value of about 0.04. These indicate that the binding of antibody 1B4 to B7-H3 protein is specific, and that the antibody has no ability to bind to other members of the B7 family.

Example 5: Construction of CAR Structures

The CAR structures used in this example were third-generation CAR structures. As shown in FIG. 7, the specific structures were as follows: at the 5' end was an scFv sequence followed by a CD8 hinge region, a CD8 transmembrane region, CD28 and 4-1BB intracellular co-stimulatory domains, and a CD3ζ signal transduction domain in sequence. Starting from the 5' end, the scFv was as follows: the heavy chain variable region of an antibody, a flexible linker polypeptide of GGGGS GGGGS GGGGS, and the light chain variable region of the antibody. Following the CAR structures, a truncated EGFR was linked by a P2A cleavage peptide.

The sequence of a CAR structure constructed using antibody 1B4 (SEQ ID NO: 27):

```
ATGGAGACAGACACTCCTGCTATGGGTGCTGCTGCTCTGGGTTCCAGG
TTCCACAGGTGACGAGGTGCAGCTGCAACAGAGCGGTGCCGAGCTGGTG
AAGCCCGGCGCGTCCGTTAAGCTATCATGCAAAGCGTCCGGCTACACGTT
CACCAACTATTGGATTAATTGGATCCGGCAGAGGCCCGGACAGGGCCTCG
AATGGATCGGTCGCATCGCGCCCGGGACGATTTCCACTTATTACAACGAG
AAATTTAAGGGCCGCGCCACCATAACTGAGGATACCTCGACCAACACCGC
CTACCTGCAGCTTTCGTCCCTGACCTCGGAGGACACGGCCGTGTACTTCTG
TGCTCGCCAGGACAACTACTTTATTAACTGGGGCCAGGGTACTCTTGTCA
CCGTCAGCTCGGGGGGCGGCGGCTCCGGAGGCGGTGGTTCAGGCGGGGG
```

AGGCTCCGACATCGTGCTGACCCAGAGCCCTGCTTCCTTGAGCGCATCTC
TGGGCGAGAAGGTAACTATCACCTGCTCTGCTTCGTCCTCTATCTCTTCTA
GTGACCTGCACTGGTACCAGCAGAAGTCCGGTACAAGTCCTCGTCCGTGG
ATCTACGGCACCAGCAACCTGGCCTCTGGCGTCCCACCGCGCTTCTCTGG
AAGCGGGTCCGGGACTTCCTTTTCGCTCACAATCAGCTCCGTGGAGGCTG
AAGATGTGGGCACCTACTACTGTCAGCAGTGGTTCAGCTACCCCTTCACC
TTCGGCACCGGCACCAAGCTGGAGATCAAGGCGGCCGCATTCGTGCCGGT
CTTCCTGCCAGCGAAGCCCACCACGACGCCAGCGCCGCGACCACCAACAC
CGGCGCCCACCATCGCGTCGCAGCCCTGTCCCTGCGCCCAGAGGCGTGC
CGGCCAGCGGCGGGGGGCGCAGTGCACACGAGGGGGCTGGACTTCGCCT
GTGATATCTACATCTGGGCGCCCTTGGCCGGGACTTGTGGGGTCCTTCTCC
TGTCACTGGTTATCACCCTTTACTGCAACCACAGGAACAGGAGTAAGAGG
AGCAGGCTCCTGCACAGTGACTACATGAACATGACTCCCCGCCGCCCCGG
GCCCACCCGCAAGCATTACCAGCCCTATGCCCCACCACGCGACTTCGCAG
CCTATCGCTCCCGTTTCTCTGTTGTTAAACGGGGCAGAAAGAAGCTCCTGT
ATATATTCAAACAACCATTTATGAGACCAGTACAAACTACTCAAGAGGAA
GATGGCTGTAGCTGCCGATTTCCAGAAGAAGAAGAAGGAGGATGTGAAC
TGAGAGTGAAGTTCAGCAGGAGCGCAGACGCCCCGCGTACCAGCAGGG
CCAGAACCAGCTCTATAACGAGCTCAATCTAGGACGAAGAGAGGAGTAC
GATGTTTTGGACAAGAGACGTGGCCGGGACCCTGAGATGGGGGGAAAGC
CGAGAAGGAAGAACCCTCAGGAAGGCCTGTACAATGAACTGCAGAAAGA
TAAGATGGCGGAGGCCTACAGTGAGATTGGGATGAAAGGCGAGCGCCGG
AGGGGCAAGGGGCACGATGGCCTTTACCAGGGTCTCAGTACAGCCACCA
AGGACACCTACGACGCCTTCACATGCAGGCCTGCCCCCTCGC

The sequence of a CAR structure constructed using antibody 2Y31 (SEQ ID NO: 28):

ATGGAGACAGACACACTCCTGCTATGGGTGCTGCTGCTCTGGGTTCCAGG
TTCCACAGGTGACGAGTTCGAGGTGCAGCTGGTGGAGAGCGGAGGTGGG
CTAGTTAAACCCGGCGGCTCCTTGAAGCTGTCATGCGCTGCTTCTGGGTTT
GCGTTCTCCCGCTACGACATGAGTTGGGTGCGCCAGAGCCCTGAAAAGCG

CCTGGAGTGGGTCGCCACCATTAGCGATGACGGCAGACACACCTACGAC

AGGGATAGCGTAAAGGGTCGCTTCACCATCTCTCGTGACAACGCCAAGAA

CACGCTTTACCTGCAGATGTCCTCTCTGCGCTCGGAGGACACCGCGCTCT

ACTACTGCGTGCGACATCGCGCCATCACTACTGCACGGTTCGACTATTGG

GGCCAGGGCACCACAGTCACCGTGTCGTCCTCCCGTGGGGGCGGCGGCTC

CGGAGGCGGTGGTTCAGGCGGGGGAGGCTCCGGGTGCGCGGACATCGTG

ATGACCCAGAGTCCGTCGTCTCTGAGCGTCTCGCTCGGCGACACCGTGAG

CATCACTTGTAAAGCTTCCCAGGACATCTACTCCAACATCGGTTGGCTAC

AACAGCTGCCCGGACAGTCCTTCAAGGGCCTGATTTACCACGGGACCAAC

CTGGAGGACGGCGTTCCTTCCCGCTTCAGCGGCTCCGGCTCCGGTACAGA

TTACTCTCTGACCATTTCTGGCCTTGAGAGCGAAGACTTTGCCGATTACTA

CTGCCTGCAGTACGTGCAGTTCCCCTATACCTTCGGCGGTGGCACTAAGTT

GGAGATCAAGGCCTCAGGCGCGGCCGCATTCGTGCCGGTCTTCCTGCCAG

CGAAGCCCACCACGACGCCAGCGCCGCGACCACCAACACCGGCGCCCAC

CATCGCGTCGCAGCCCCTGTCCCTGCGCCCAGAGGCGTGCCGGCCAGCGG

CGGGGGGCGCAGTGCACACGAGGGGGCTGGACTTCGCCTGTGATATCTAC

ATCTGGGCGCCCTTGGCCGGGACTTGTGGGGTCCTTCTCCTGTCACTGGTT

ATCACCCTTTACTGCAACCACAGGAACAGGAGTAAGAGGAGCAGGCTCC

TGCACAGTGACTACATGAACATGACTCCCCGCCGCCCCGGGCCCACCCGC

AAGCATTACCAGCCCTATGCCCCACCACGCGACTTCGCAGCCTATCGCTC

CCGTTTCTCTGTTGTTAAACGGGGCAGAAAGAAGCTCCTGTATATATTCA

AACAACCATTTATGAGACCAGTACAAACTACTCAAGAGGAAGATGGCTG

TAGCTGCCGATTTCCAGAAGAAGAAGAAGGAGGATGTGAACTGAGAGTG

AAGTTCAGCAGGAGCGCAGACGCCCCGCGTACCAGCAGGGCCAGAACC

AGCTCTATAACGAGCTCAATCTAGGACGAAGAGGAGTACGATGTTTTG

GACAAGAGACGTGGCCGGGACCCTGAGATGGGGGGAAAGCCGAGAAGG

AAGAACCCTCAGGAAGGCCTGTACAATGAACTGCAGAAAGATAAGATGG

CGGAGGCCTACAGTGAGATTGGGATGAAAGGCGAGCGCCGGAGGGGCAA

GGGGCACGATGGCCTTTACCAGGGTCTCAGTACAGCCACCAAGGACACCT

ACGACGCCCTTCACATGCAGGCCCTGCCCCCTCGC

Example 6: Assay for Ability of CAR-B7-H3-Expressing Jurkat T Cells to Bind to B7-H3 Protein

The B7-H3 and CAR-B7-H3 binding specificwas assessed by flow cytometry. Specifically, CAR-B7-H3 was stably expressed on Jurkat T cells. In a 100 μL flow cytometry buffer staining system, biotin-B7-H3 antigen, at different concentrations (ranging from 0.03125 μg/mL to 16 μg/mL), was incubated with $1 \times 10^5$ CAR-B7-H3-Jurkat T cells at 4 °C for 45 min. After the incubation, the mixture was centrifuged at 500 g for 5 min, and the supernatant was discarded. After the cells were resuspended in 500 μL of flow cytometry buffer, the suspension was centrifuged at 500 g for 5 min, and the supernatant was discarded. The cells were resuspended in 100 μL of flow cytometry buffer, and 0.5 μL of fluorescently labeled streptavidin secondary antibody and 0.5 μL of fluorescently labeled anti-human EGFR antibody were added simultaneously. The mixture was incubated at 4 °C for 30 min. After the incubation, the mixture was centrifuged at 500 g for 5 min, and the supernatant was discarded. After the cells were resuspended in 300 μL of flow cytometry buffer, the fluorescence signals of the streptavidin secondary antibody in the EGFR-positive population were analyzed by flow cytometry.

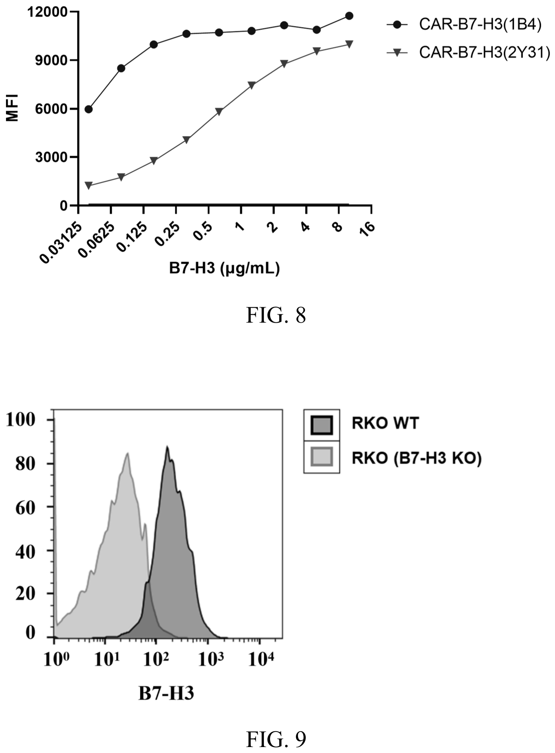

The results are shown in FIG. 8. It can be seen from the results that both CAR-B7-H3 (1B4) and CAR-B7-H3 (2Y31) exhibited abilities to bind to B7-H3 protein, with the ability of CAR-B7-H3 (1B4) to bind to B7-H3 being higher.

Example 7: Functional Assay of CAR-B7-H3 (1B4)-Expressing Jurkat T Cells

A B7-H3 knockout RKO tumor cell strain was constructed using gene editing technology and verified by flow cytometry using antibody 1B4. The results are shown in FIG. 9: the wild-type RKO tumor cells (dark peak, RKO WT) expressed high levels of B7-H3, and the B7-H3 knockout RKO tumor cells did not express B7-H3 (light peak, RKO B7-H3 KO).

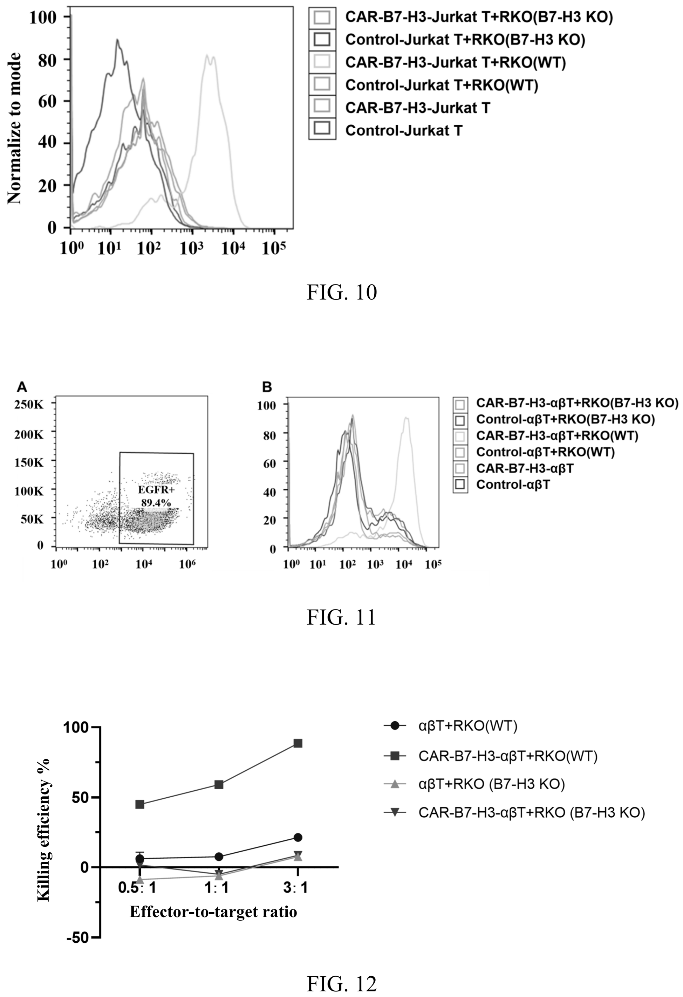

Subsequently, the CD69 signal activation level of CAR-B7-H3 was further determined. Effector cells (including Jurkat T-control cells not expressing CAR-B7-H3 and CAR-B7-H3-expressing CAR-B7-H3-Jurkat T cells) were incubated with tumor cells (RKO WT and RKO B7-H3 KO) in an effector-to-target ratio of 1:1. Specifically, the incubation was performed in a 24-well plate, with $0.5 \times 10^6$ effector cells and $0.5 \times 10^6$ tumor cells added. After 24 h of incubation, flow cytometry analysis was performed. The flow cytometry staining system was: anti-human EGFR and anti-human CD69. The results are shown in FIG. 10. It can be seen from the results that: after CAR-B7-H3 (1B4)-expressing Jurkat T (CAR-B7-H3-Jurkat T) cells were stimulated by wild-type RKO (RKO WT) tumor cells, their CD69 signal was significantly up-regulated, while no CD69 signal enhancement was observed with B7-H3-deficient RKO tumor cells. Furthermore, the expression of CAR-B7-H3 (1B4) did not up-regulate the background signal of CD69, precluding its activating toxic effects on T cells.

Example 8: CAR Transduction Positivity Rate and Functional Assay of CAR-B7-H3 (1B4)-Expressing αβ T Cells

(1) CAR transduction positivity rate

CAR-B7-H3 (1B4)-αβ T cells were labeled and stained with a fluorescently labeled EGFR antibody and assayed using a flow cytometer. The results are shown in FIG. 11A. It can be seen from the results that the EGFR positivity rate was 89.4%, which means that the CAR transduction positivity rate was 89.4%.

(2) Expression levels of CD69 of CAR-B7-H3 (1B4)-αβ T cells

To analyze the expression levels of CD69 of CAR-B7-H3 (1B4)-αβ T cells, effector cells (including αβ T-control cells not expressing CAR-B7-H3 and CAR-B7-H3-expressing CAR-B7-H3-αβ T cells) were incubated with tumor cells (RKO WT and RKO B7-H3 KO) in an effector-to-target ratio of 1:1. Specifically, the incubation was performed in a 24-well plate, with $0.5 \times 10^6$ effector cells and $0.5 \times 10^6$ tumor cells added. After 24 h of incubation, flow cytometry analysis was performed. The flow cytometry staining system was: anti-human CD3, anti-human αβ TCR, anti-human EGFR, and anti-human CD69.

The results are shown in FIG. 11B: RKO WT tumor cells significantly increased CD69 expression in CAR-B7-H3 (1B4)-αβ T cells, while B7-H3-deficient RKO tumor cells did not affect CD69 expression in CAR-B7-H3 (1B4)-αβ T cells. Moreover, after expressing CAR-B7-H3, αβ T cells did not cause the up-regulation of their own CD69 background signal.

(3) Ability of CAR-B7-H3 (1B4)-αβ T cells to kill RKO tumor cells *in vitro*

The *in vitro* cell killing assay was based on the luciferase fluorescence detection method. The tumor cells to be tested (including RKO WT and B7-H3 knockout RKO cells (RKO B7-H3 KO)) stably expressed luciferase. Killing tests were performed in a 24-well plate, with different effector-to-target ratios (0.5:1, 1:1, and 3:1) set in this assay. For the effector-to-target ratio of 0.5:1, tumor cells and effector cells were counted separately, and the two types of cells were resuspended in their respective culture media: effector cells were resuspended to $0.25 \times 10^6$ cells/mL, and tumor cells were resuspended to $0.5 \times 10^6$ cells/mL. For the effector-to-target ratio of 1:1, both effector cells and tumor cells were resuspended to $0.5 \times 10^6$ cells/mL. For the effector-to-target ratio of 3:1, effector cells were resuspended to $1.5 \times 10^6$ cells/mL, and tumor cells were resuspended to $0.5 \times 10^6$ cells/mL. 500 μL of tumor cells and 500 μL of effector cells were added to the same well and mixed thoroughly and homogeneously. A control well containing only tumor cells was set, with 500 μL of tumor cell culture medium and 500 μL of effector cell culture medium added. The plate was incubated in a cell incubator. After 24 h, luciferase fluorescence signals were detected.

$$\text{Killing efficiency (cytotoxicity\%)} = \frac{\text{Control well signal value - Test well signal value}}{\text{Control well signal value}} \times 100\%$$

The results are shown in FIG. 12. It can be seen from the results that compared to the control $\alpha\beta$ T cells ($\alpha\beta$ T), CAR-B7-H3-expressing $\alpha\beta$ T cells exhibited a very good killing effect on RKO tumor cells *in vitro,* and their killing ability gradually increased as the effector-to-target ratio increased; however, they exhibited no killing effect on B7-H3-deficient RKO tumor cells. This can also further verify that the killing effect of CAR-B7-H3-$\alpha\beta$ T cells on RKO tumor cells was based on B7-H3 recognition.

(4) Ability of CAR-B7-H3 (1B4)-$\alpha\beta$ T cells to kill glioma cells *in vitro*

In this patent, the ability of CAR-B7-H3-expressing $\alpha\beta$ T cells to kill glioma tumor cells (including HTB15 and TJ905) was further assessed using the same experimental method as in (3).

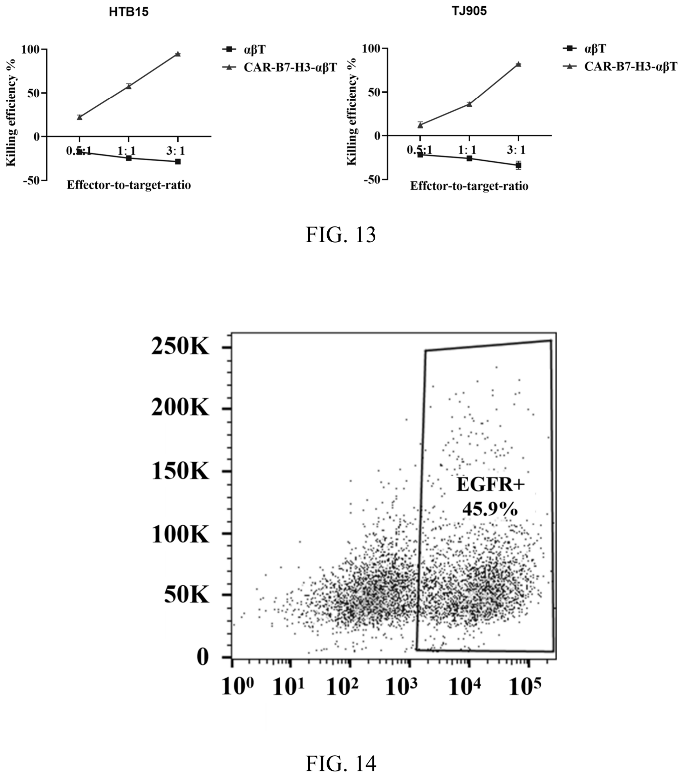

The results are shown in FIG. 13. It can be seen from the results that CAR-B7-H3-expressing $\alpha\beta$ T cells also exhibited very strong killing effects on glioma tumor cells (including HTB15 and TJ905), and their killing ability gradually increased as the effector-to-target ratio increased. By contrast, $\alpha\beta$ T cells not expressing CAR-B7-H3 ($\alpha\beta$ T) exhibited no ability to kill these two strains of glioma tumor cells. This can also further verify that the killing effects of CAR-P7-H3-$\alpha\beta$ T cells on these two strains of glioma tumor cells were based on B7-H3 recognition.

Example 9: CAR Transduction Positivity Rate and Functional Assay of CAR-B7-H3 (1B4)-Expressing $\gamma\delta$ T Cells

(1) CAR transduction positivity rate

CAR-B7-H3 (1B4)-$\gamma\delta$ T cells were labeled and stained with a fluorescently labeled EGFR antibody and assayed using a flow cytometer. The results are shown in FIG. 14. It can be seen from the results that the EGFR positivity rate was 45.9%, which means that the CAR transduction positivity rate was 45.9%.

(2) Killing effects of CAR-B7-H3 (1B4)-expressing CAR-$\alpha\beta$ T and CAR-$\gamma\delta$ T on RKO WT tumor cells

The killing effects of CAR-B7-H3-expressing CAR-$\alpha\beta$ T and CAR-$\gamma\delta$ T on RKO WT tumor cells were further analyzed and compared using the experimental method in (3) of Example 8.

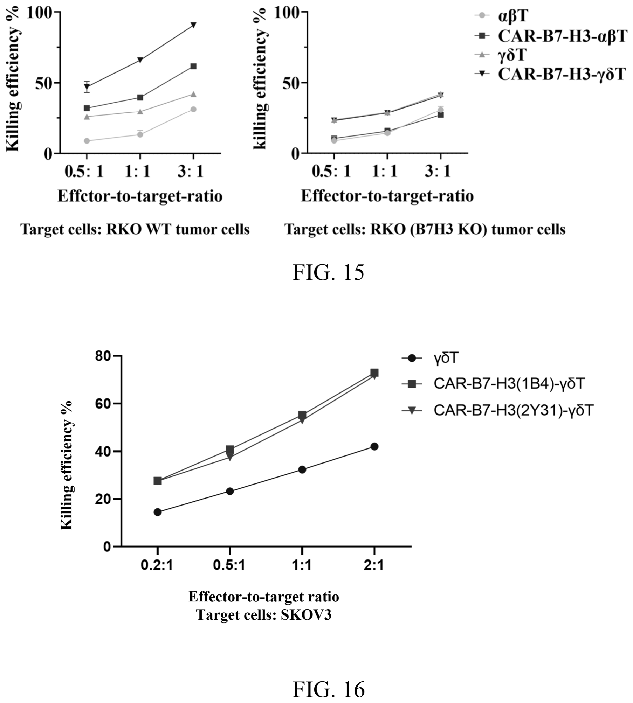

The results are shown in FIG. 15. It can be seen from the results that: the killing ability of CAR-B7-H3-$\alpha\beta$ T cells was significantly higher than that of $\alpha\beta$ T cells not expressing CAR-B7-H3 (FIG. 15A), and the ability of CAR-B7-H3-$\alpha\beta$ T cells to kill B7-H3 knockout RKO tumor cells was comparable to that of $\alpha\beta$ T cells not expressing CAR-B7-H3 (FIG. 15B); the ability of CAR-B7-H3-$\gamma\delta$ T cells to kill RKO tumor cells was significantly higher than that of $\gamma\delta$ T cells not expressing CAR-B7-H3 (FIG. 15A), and the ability of CAR-B7-H3-$\gamma\delta$ T cells to kill B7-H3 knockout RKO tumor cells was comparable to that of $\gamma\delta$ T cells not expressing CAR-B7-H3 (FIG. 15B). This further verifies that CAR-B7-H3-$\alpha\beta$ T cells and CAR-B7-H3-$\gamma\delta$ T cells enhance the killing of RKO tumor cells by recognizing B7-H3.

In addition, it can also be seen that the ability of CAR-B7-H3-$\gamma\delta$ T cells to kill RKO tumor cells was significantly higher than that of CAR-B7-H3-$\alpha\beta$ T cells under conditions of the same transduction positivity rate (the transduction positivity rate of CAR-B7-H3-$\alpha\beta$ T cells was lowered to be consistent with that of CAR-B7-H3-$\gamma\delta$ T cells by adding $\alpha\beta$ T cells not expressing the CAR to CAR-B7-H3-$\alpha\beta$ T cells) and the same effector-to-target ratio.

Moreover, $\gamma\delta$ T cells not expressing CAR-B7-H3 also exhibited a certain ability to kill RKO tumor cells (FIG. 15A), and the killing ability was significantly higher than that of $\alpha\beta$ T cells not expressing CAR-B7-H3. This further indicates that the anti-tumor ability of $\gamma\delta$ T cells was higher than that of $\alpha\beta$ T cells.

Example 10: Examination of Functional Differences Between Two Types of CAR-γδ T Cells Derived from Different Antibodies

The killing effects of γδ T cells expressing CAR-B7-H3 (1B4) and CAR-B7-H3 (2Y31) on RKO WT tumor cells were further analyzed and compared using the experimental method in (3) of Example 8.

The results are shown in FIG. 16. It can be seen from the results that, whether the scFv was derived from antibody 1B4 or 2Y31, CAR-B7-H3-γδ T cells exhibited an ability to kill SKOV3, and the killing ability was significantly higher than that of unmodified γδ T cells. The abilities of the two types of CAR-B7-H3-γδ T cells derived from different antibodies to kill SKOV3 did not significantly differ. Under all killing conditions, the anti-tumor abilities of both types of CAR-B7-H3-γδ T cells derived from different antibodies increased as the effector-to-target ratio increased.

Example 11: *In Vivo* Anti-Tumor Ability Assay

In this assay, an animal model of intraperitoneal tumor formation was used. Each mouse was intraperitoneally inoculated with $1 \times 10^6$ SKOV3 tumor cells (stably expressing luciferase): the $1 \times 10^6$ SKOV3 tumor cells were resuspended in 200 μL of PBS and intraperitoneally injected (the day of inoculation was taken as day 0 post-inoculation). On day 4 post-SKOV3 tumor cell inoculation, body weight measurements and *in vivo* tumor imaging analysis were performed. Mice were randomized into 3 groups according to data on body weight and tumor size: a control group, a γδ T treatment group, and a CAR-B7-H3 (1B4)-γδ T treatment group.

On day 5 post-inoculation, cell intervention treatment was performed: 200 μL of PBS was intraperitoneally injected into each mouse in the control group, $1 \times 10^6$ γδ T cells (resuspended in 200 μL of PBS) were intraperitoneally injected into each mouse in the γδ T treatment group, and $1 \times 10^6$ CAR-B7-H3 (1B4)-γδ T cells (resuspended in 200 μL of PBS) were intraperitoneally injected into each mouse in the CAR-B7-H3-γδ T treatment group. Subsequently, *in vivo* tumor imaging analysis was performed regularly, observations were performed on the survival of the mice, and survival curves were statistically analyzed.

The *in vivo* tumor imaging procedure: A mouse was anesthetized with an isoflurane anesthesia machine, and 100 μL of luciferin substrate (2 mg) was drawn with an insulin syringe and then intraperitoneally injected into the mouse. After 10 min, imaging was performed using an IVIS fluorescence imaging system. The images were saved, and fluorescence values were statistically analyzed.

Experimental results

(1) Tumor growth in mice

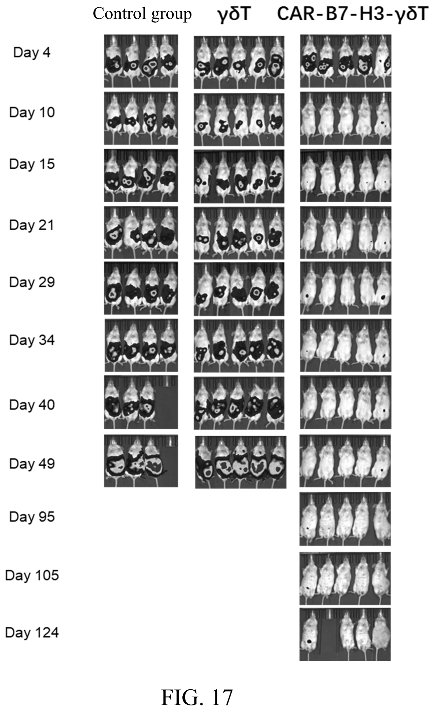

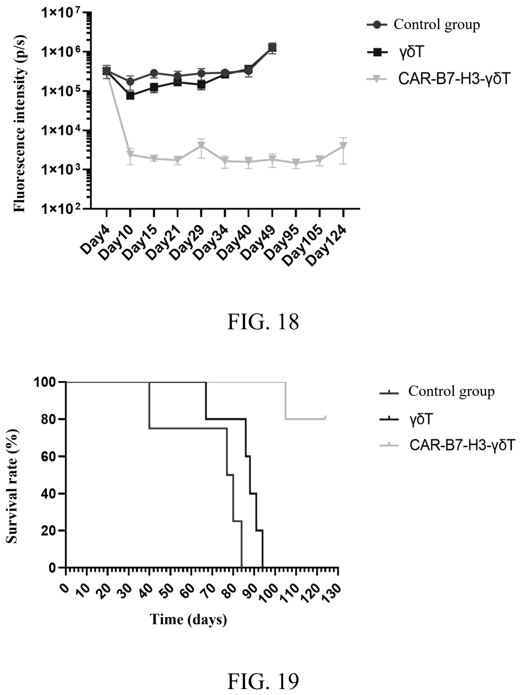

Referring to FIGs. 17 and 18, it can be seen that, for the SKOV3 tumor model of intraperitoneal tumor formation, intraperitoneal reinfusion of CAR-B7-H3-γδ T significantly inhibited tumor growth. Unmodified γδ T cells exhibited a certain ability to inhibit tumors; however, the effect was not significant.

(2) Survival of tumor model mice

Referring to FIG. 19, it can be seen that: the tumor-bearing mice in the control group all died at about 85 days; the survival time of the mice in the γδ T treatment group was extended by about 10 days, reaching up to 95 days; the survival time of the mice in the CAR-B7-H3-γδ T treatment group significantly increased, and a survival rate of 80% was maintained even at 125 days.

**Claims**

1. An isolated antibody or antibody fragment that specifically binds to B7-H3, comprising a heavy chain variable region VH and a light chain variable region VL, wherein the heavy chain variable region VH comprises: a VH-CDR1 represented by the amino acid sequence of SEQ ID NO: 9, a VH-CDR2 represented by the amino acid sequence of SEQ ID NO: 10, and a VH-CDR3 represented by the amino acid sequence of SEQ ID NO: 11; the light chain variable region VL comprises: a VL-CDR1 represented by the amino acid sequence of SEQ ID NO: 12, a VL-CDR2 represented by the amino acid sequence of SEQ ID NO: 13, and a VL-CDR3 represented by the amino acid sequence of SEQ ID NO: 14.

2. An isolated antibody or antibody fragment that specifically binds to B7-H3, comprising a heavy chain variable region VH and a light chain variable region VL, wherein the heavy chain variable region VH comprises a VH-CDR1 represented by the amino acid sequence of SEQ ID NO: 15, a VH-CDR2 represented by the amino acid sequence of SEQ ID NO: 16, and a VH-CDR3 represented by the amino acid sequence of SEQ ID NO: 17; the light chain variable region VL comprises: a VL-CDR1 represented by the amino acid sequence of SEQ ID NO: 18, a VL-CDR2 represented by the amino acid sequence of SEQ ID NO: 19, and a VL-CDR3 represented by the amino acid sequence of SEQ ID NO: 20.

3. The isolated antibody or antibody fragment that specifically binds to B7-H3 as claimed in claim 1 or 2, wherein: the heavy chain variable region VH is selected from any one of: the amino acid sequence set forth in SEQ ID NO: 1; an amino acid sequence having 80% or more, or 85% or more, or 88% or more, or 90% or more, or 93% or more, or 95% or more, or 98% or more identity to the amino acid sequence set forth in SEQ ID NO: 1 outside the CDRs; the amino acid sequence set forth in SEQ ID NO: 2; and an amino acid sequence having 80% or more, or 85% or more, or 88% or more, or 90% or more, or 93% or more, or 95% or more, or 98% or more identity to the amino acid sequence set forth in SEQ ID NO: 2 outside the CDRs.

4. The isolated antibody or antibody fragment that specifically binds to B7-H3 as claimed in claim 1 or 2, wherein: the light chain variable region VL is selected from any one of: the amino acid sequence set forth in SEQ ID NO: 3; an amino acid sequence having 80% or more, or 85% or more, or 88% or more, or 90% or more, or 93% or more, or 95% or more, or 98% or more identity to the amino acid sequence set forth in SEQ ID NO: 3 outside the CDRs; the amino acid sequence set forth in SEQ ID NO: 4; and an amino acid sequence having 80% or more, or 85% or more, or 88% or more, or 90% or more, or 93% or more, or 95% or more, or 98% or more identity to the amino acid sequence set forth in SEQ ID NO: 4 outside the CDRs.

5. An isolated antibody or antibody fragment that specifically binds to B7-H3, comprising:

   (i) a heavy chain variable region VH represented by SEQ ID NO: 1 and a light chain variable region VL represented by SEQ ID NO: 3, or
   (ii) a heavy chain variable region VH represented by SEQ ID NO: 2 and a light chain variable region VL represented by SEQ ID NO: 4.

6. The isolated antibody or antibody fragment that specifically binds to B7-H3 as claimed in any one of claims 1-5, wherein the antibody or antibody fragment is a genetically engineered antibody or antibody fragment.

7. The isolated antibody or antibody fragment that specifically binds to B7-H3 as claimed in any one of claims 1-6, wherein the antibody or antibody fragment is an scFv.

8. A chimeric antigen receptor, comprising: the antibody or antibody fragment that specifically binds to B7-H3 as claimed in claim 7.

9. The chimeric antigen receptor as claimed in claim 8, comprising: the antibody or antibody fragment as claimed in claim 7, and a transmembrane region fused to a carboxyl-terminus of the antibody or antibody fragment.

10. The chimeric antigen receptor as claimed in claim 8 or 9, comprising: the antibody or antibody fragment as claimed in claim 7, a transmembrane region fused to a carboxyl-terminus of the antibody or antibody fragment, and an immunocompetent cell activation signal transduction region fused to the carboxyl-terminus of the transmembrane region.

11. The chimeric antigen receptor as claimed in any one of claims 8-10, further comprising any one or any two or more of a membrane protein, a secreted protein, an intracellular protein, a small-molecule drug, and a cytotoxic drug.

12. A nucleic acid molecule, comprising: a nucleotide sequence encoding the antibody or antibody fragment as claimed in any one of claims 1-7 or the chimeric antigen receptor as claimed in any one of claims 8-11.

13. The nucleic acid molecule as claimed in claim 12, comprising:

   (i) the nucleotide sequence set forth in SEQ ID NO: 5, which encodes the heavy chain variable region, and the nucleotide sequence set forth in SEQ ID NO: 7, which encodes the light chain variable region, or
   (ii) the nucleotide sequence set forth in SEQ ID NO: 6, which encodes the heavy chain variable region, and the nucleotide sequence set forth in SEQ ID NO: 8, which encodes the light chain variable region.

14. A vector, comprising the nucleic acid molecule as claimed in claim 12 or 13.

15. The vector as claimed in claim 14, wherein the vector is a lentiviral vector, a retroviral vector, an adenoviral vector, or an adeno-associated virus vector.

16. A cell, comprising the nucleic acid molecule as claimed in claim 12 or 13 or the vector as claimed in claim 14 or 15.

17. The cell as claimed in claim 16, wherein the cell includes autologous or allogeneic T cells, B cells, NK cells, macrophages, monocytes, dendritic cells, neutrophils, basophils, eosinophils, mast cells, NK-T cells, MAIT cells, hematopoietic stem cells, embryonic stem cells, induced pluripotent stem cells, and erythrocytes; the T cells include $\alpha\beta$ T cells, $\gamma\delta$ T cells, and regulatory T cells.

18. A pharmaceutical composition,

   comprising: any one or more selected from the antibody or antibody fragment as claimed in any one of claims 1-7, the chimeric antigen receptor as claimed in any one of claims 8-11, the nucleic acid molecule as claimed in claim 12 or 13, the vector as claimed in claim 14 or 15, and the cell as claimed in claim 16 or 17, or
   comprising: any one or more selected from the antibody or antibody fragment as claimed in any one of claims 1-7, the chimeric antigen receptor as claimed in any one of claims 8-11, the nucleic acid molecule as claimed in claim 12 or 13, the vector as claimed in claim 14 or 15, and the cell as claimed in claim 16 or 17, and a pharmaceutically acceptable carrier, or
   comprising: any one or more selected from the antibody or antibody fragment as claimed in any one of claims 1-7, the chimeric antigen receptor as claimed in any one of claims 8-11, the nucleic acid molecule as claimed in claim 12 or 13, the vector as claimed in claim 14 or 15, and the cell as claimed in claim 16 or 17, and another pharmaceutically active agent or drug.

19. A method of treatment or prevention of B7-H3-positive diseases, comprising administering to a subject a therapeutically effective amount of the antibody or antibody fragment as claimed in any one of claims 1-7, the chimeric antigen receptor as claimed in any one of claims 8-11, the nucleic acid molecule as claimed in claim 12 or 13, the vector as claimed in claim 14 or 15, the cell as claimed in claim 16 or 17, or the pharmaceutical composition as claimed in claim 18.

20. The method as claimed in claim 19, wherein the B7-H3-positive diseases include malignant/benign hematological tumors, malignant/benign solid tumors, autoimmune diseases, bacterial infections, viral infections, parasitic infections, abnormal bone growth, allogeneic transplantation, and transplant rejection; the autoimmune diseases include systemic lupus erythematosus, rheumatoid arthritis, multiple sclerosis, Sjogren's syndrome, and ankylosing spondylitis.

21. The method as claimed in claim 20, wherein the B7-H3-positive diseases include acute myeloid leukemia, chronic myeloid leukemia, acute lymphocytic leukemia, non-Hodgkin lymphoma, multiple myeloma, melanoma, lung cancer, colorectal cancer, renal tumors, bladder cancer, gastrointestinal cancer, prostate cancer, liver cancer, ovarian cancer, pancreatic cancer, endometrial cancer, gastric cancer, prostate cancer, kidney cancer, cervical cancer, thyroid cancer, uterine cancer, neuroendocrine cancer, head and neck cancer, nasopharyngeal carcinoma, testicular cancer, basal cell skin cancer, squamous cell skin cancer, dermatofibrosarcoma protuberans, Merkel cell carcinoma, glioblastoma, glioma, sarcomas, mesothelioma, or myelodysplastic syndrome.

22. The antibody or antibody fragment as claimed in any one of claims 1-7, the chimeric antigen receptor as claimed in any one of claims 8-11, the nucleic acid molecule as claimed in claim 12 or 13, the vector as claimed in claim 14 or 15, the cell as claimed in claim 16 or 17, or the pharmaceutical composition as claimed in claim 18 for use in the treatment or prevention of B7-H3-positive diseases.

23. The antibody or antibody fragment, the chimeric antigen receptor, the nucleic acid molecule, the vector, the cell, or the pharmaceutical composition for use in the treatment or prevention of B7-H3-positive diseases as claimed in claim 22, wherein the B7-H3-positive diseases include malignant/benign hematological tumors, malignant/benign solid tumors, autoimmune diseases, bacterial infections, viral infections, parasitic infections, abnormal bone growth, allogeneic transplantation, and transplant rejection; the autoimmune diseases include systemic lupus erythematosus, rheumatoid arthritis, multiple sclerosis, Sjogren's syndrome, and ankylosing spondylitis.

24. The antibody or antibody fragment, the chimeric antigen receptor, the nucleic acid molecule, the vector, the cell, or the pharmaceutical composition for use in the treatment or prevention of B7-H3-positive diseases as claimed in claim 23, wherein the B7-H3-positive diseases include acute myeloid leukemia, chronic myeloid leukemia, acute lymphocytic leukemia, non-Hodgkin lymphoma, multiple myeloma, melanoma, lung cancer, colorectal cancer, renal tumors, bladder cancer, gastrointestinal cancer, prostate cancer, liver cancer, ovarian cancer, pancreatic cancer, endometrial cancer, gastric cancer, prostate cancer, kidney cancer, cervical cancer, thyroid cancer, uterine cancer, neuroendocrine cancer, head and neck cancer, nasopharyngeal carcinoma, testicular cancer, basal cell skin cancer, squamous cell skin cancer, dermatofibrosarcoma protuberans, Merkel cell carcinoma, glioblastoma, glioma, sarcomas, mesothelioma, or myelodysplastic syndrome.

25. A method for detecting B7-H3, comprising: a step of using the antibody or antibody fragment as claimed in any one of claims 1-7, the chimeric antigen receptor as claimed in any one of claims 8-11, or the nucleic acid molecule as claimed in claim 12 or 13 to detect B7-H3.

26. A kit for detection of B7-H3, comprising the antibody or antibody fragment as claimed in any one of claims 1-7 or a labeled form thereof, the chimeric antigen receptor as claimed in any one of claims 8-11 or a labeled form thereof, or the nucleic acid molecule as claimed in claim 12 or 13 or a labeled form thereof.

27. An isolation kit for isolating B7-H3-positive cells, comprising the antibody or antibody fragment as claimed in any one of claims 1-7 or a labeled form thereof, the chimeric antigen receptor as claimed in any one of claims 8-11 or a labeled form thereof, or the nucleic acid molecule as claimed in claim 12 or 13 or a labeled form thereof.

28. A method for enhancing cell function *in vitro,* comprising: a step of contacting cells with the antibody or antibody fragment as claimed in any one of claims 1-7 or a labeled form thereof, the chimeric antigen receptor as claimed in any one of claims 8-11 or a labeled form thereof, or the nucleic acid molecule as claimed in claim 12 or 13 or a labeled form thereof.

29. Use of the antibody or antibody fragment as claimed in any one of claims 1-7 for the manufacturing of a product for detecting B7-H3 protein.

30. Use of the antibody or antibody fragment as claimed in any one of claims 1-7 for the manufacturing of a product for blocking B7-H3 protein.

**A**                                    **1B4 VH**

GAAGTGCAGCTCCAGCAGAGCGGAGCAGAACTGGTGAAGCCAGGAGCCAGCGTGAAGCTGT
CTTGCAAGGCCAGCGGCTACACCTTCACCAACTACTGGATCAATTGGATCAGACAGAGGCCAG
GACAGGGACTCGAGTGGATTGGCAGAATCGCCCCAGGCACCATCAGCACCTACTACAACGAG
AAGTTCAAGGGCAGGGCCACCATCACCGAGGATACCAGCACCAACACCGCCTACCTCCAGCT
GTCTAGCCTGACAAGCGAGGACACAGCCGTGTACTTTTGCGCCAGGCAGGACAACTACTTCAT
CAATTGGGGCCAGGGAACCCTGGTGACAGTGTCCAGC

**B**                                    **2Y31 VH**

GAGTTCGAGGTGCAGCTGGTGGAGAGCGGAGGTGGGCTAGTTAAACCCGGCGGCTCCTTGAA
GCTGTCATGCGCTGCTTCTGGGTTTGCGTTCTCCCGCTACGACATGAGTTGGGTGCGCCAGAGC
CCTGAAAAGCGCCTGGAGTGGGTCGCCACCATTAGCGATGACGGCAGACACACCTACGACAG
GGATAGCGTAAAGGGTCGCTTCACCATCTCTCGTGACAACGCCAAGAACACGCTTTACCTGCA
GATGTCCTCTCTGCGCTCGGAGGACACCGCGCTCTACTACTGCGTGCGACATCGCGCCATCAC
TACTGCACGGTTCGACTATTGGGGCCAGGGCACCACAGTCACCGTGTCGTCCTCCCGT

## FIG. 1

**A**                                    **1B4 VL**

GACATTGTTCTTACACAATCTCCGGCCAGCCTCTCTGCGAGCCTCGGGGAAAAGGTGACAATC
ACTTGTTCCGCATCATCAAGTATCTCCAGCTCTGACCTGCACTGGTATCAGCAGAAAAGTGGCA
CCAGTCCGAGGCCGTGGATCTATGGCACTAGCAATCTTGCGTCAGGAGTCCCACCCCGCTTTA
GTGGCAGTGGATCAGGGACATCATTCAGTCTGACAATCTCTAGCGTCGAGGCGGAGGATGTCG
GCACATATTACTGCCAACAGTGGTTTTCTTATCCTTTCACATTTGGTACGGGCACGAAACTGGA
AATAAAA

**B**                                    **2Y31 VL**

GGGTGCGCGGACATCGTGATGACCCAGAGTCCGTCGTCTCTGAGCGTCTCGCTCGGCGACACC
GTGAGCATCACTTGTAAAGCTTCCCAGGACATCTACTCCAACATCGGTTGGCTACAACAGCTGC
CCGGACAGTCCTTCAAGGGCCTGATTTACCACGGGACCAACCTGGAGGACGGCGTTCCTTCCC
GCTTCAGCGGCTCCGGCTCCGGTACAGATTACTCTCTGACCATTTCTGGCCTTGAGAGCGAAGA
CTTTGCCGATTACTACTGCCTGCAGTACGTGCAGTTCCCCTATACCTTCGGCGGTGGCACTAAG
TTGGAGATCAAGGCCTCAGGC

## FIG. 2

**A**                                    **1B4 VH**

EVQLQQSGAELVKPGASVKLSCKASGYTFTNYWINWIRQRPGQGLEWIGRIAPGTISTYYNEKFKGR
ATITEDTSTNTAYLQLSSLTSEDTAVYFCARQDNYFINWGQGTLVTVSS

**B**                                    **2Y31 VH**

EFEVQLVESGGGLVKPGGSLKLSCAASGFAFSRYDMSWVRQSPEKRLEWVATISDDGRHTYDRDSV
KGRFTISRDNAKNTLYLQMSSLRSEDTALYYCVRHRAITTARFDYWGQGTTVTVSSSR

## FIG. 3

**A**                        **1B4 VL**

DIVLTQSPASLSASLGEKVTITCSASSSISSSDLHWYQQKSGTSPRPWIYGTSNLASGVPPRFSGSGSG
TSFSLTISSVEAEDVGTYYCQQWFSYPFTFGTGTKLEIK

**B**                        **2Y31 VL**

GCADIVMTQSPSSLSVSLGDTVSITCKASQDIYSNIGWLQQLPGQSFKGLIYHGTNLEDGVPSRFSGS
GSGTDYSLTISGLESEDFADYYCLQYVQFPYTFGGGTKLEIKASG

### FIG. 4

RU

| Ka (1/Ms) | Kd (1/s) | KD (M) |
|-----------|----------|--------|
| 2.81E+05 | 7.38E-05 | 2.625E-10 |

### FIG. 5

**A**

U87

U87(B7-H3 KO)

**B**

U87

U87(B7-H3 KO)

m2Y31
hu2Y31
8H9
NC

FIG. 6

| VH-(G4S)3-VL | CD8 Hinge | CD8 TM | CD28 | 4-1BB | CD3z | EGFRt |

P2A

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

## INTERNATIONAL SEARCH REPORT

| International application No. |
| --- |
| **PCT/CN2023/142028** |

| A. | CLASSIFICATION OF SUBJECT MATTER |
| --- | --- |

C07K16/28(2006.01)i; A61K39/395(2006.01)i; A61P35/00(2006.01)i; A61P37/02(2006.01)i; A61P31/04(2006.01)i; A61P31/12(2006.01)i; A61P33/00(2006.01)i; A61P19/08(2006.01)i; A61P37/06(2006.01)i; C07K19/00(2006.01)i; C12N15/13(2006.01)i; C12N15/867(2006.01)i; C12N5/10(2006.01)i; C12N15/62(2006.01)i; G01N33/68(2006.01)i; G01N33/574(2006.01)i

According to International Patent Classification (IPC) or to both national classification and IPC

| B. | FIELDS SEARCHED |
| --- | --- |

Minimum documentation searched (classification system followed by classification symbols)

    IPC: C07K, A611K, A61P, C12N, G01N

Documentation searched other than minimum documentation to the extent that such documents are included in the fields searched

Electronic data base consulted during the international search (name of data base and, where practicable, search terms used)

    CNABS, DWPI, WPABS, CNTXT, ENTXT, USTXT, JPTXT, EPTXT, WOTXT, VEN, CNKI, 中国专利生物序列检索系统, China Patent Biological Sequence Search System, Genbank, EMBL, PubMed: 申请人, applicant, 发明人, inventor, B7-H3, B7H3, CD276, 抗体, antibody, 重链可变区, 轻链可变区, CDR, complementary determining region, 互补决定区, 嵌合抗原受体, chimeric antigen receptors, 肿瘤, 自身免疫病, 感染, 移植, tumors, autoimmune disease, infections, transplantation, SEQ ID NOs: 1-20

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| PX | CN 116041518 A (BEIJING QINGHUI LIANNUO BIOTECHNOLOGY CO., LTD.) 02 May 2023 (2023-05-02) claims 1-27 | 1-30 |
| A | CN 112961242 A (GUANGZHOU BIO-GENE TECHNOLOGY CO., LTD.) 15 June 2021 (2021-06-15) claims 1-10 | 1-30 |
| A | CN 111944050 A (SUZHOU PULEKANG PHARMACEUTICAL TECHNOLOGY CO., LTD.) 17 November 2020 (2020-11-17) claims 1-10 | 1-30 |
| A | CN 103687945 A (DAICHI SANKYO COMPANY, LTD. et al.) 26 March 2014 (2014-03-26) claims 1-41 | 1-30 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **15 March 2024** | **22 April 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/142028**

### C.    DOCUMENTS CONSIDERED TO BE RELEVANT

| Category* | Citation of document, with indication, where appropriate, of the relevant passages | Relevant to claim No. |
|---|---|---|
| A | CN 109851673 A (SUZHOU BRIGHT SCISTAR BIOTECHNOLOGY CO., LTD.) 07 June 2019 (2019-06-07)<br>     claims 1-7 | 1-30 |
| A | CN 115066436 A (DEUTSCHES KREBSFORSCHUNGSZENTRUM STIFTUNG DES OEFFENTLICHEN RECHTS et al.) 16 September 2022 (2022-09-16)<br>     claims 1-17 | 1-30 |
| A | WO 2021027674 A1 (KEYMED BIOSCIENCES CO., LTD.) 18 February 2021 (2021-02-18)<br>     claims 1-16 | 1-30 |

Form PCT/ISA/210 (second sheet) (July 2022)

**INTERNATIONAL SEARCH REPORT**

| International application No. |
| --- |
| **PCT/CN2023/142028** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2023/142028** |

**Box No. II     Observations where certain claims were found unsearchable (Continuation of item 2 of first sheet)**

This international search report has not been established in respect of certain claims under Article 17(2)(a) for the following reasons:

1. ☑ Claims Nos.: **19-21**
   because they relate to subject matter not required to be searched by this Authority, namely:

   Claims 19-21 relate to a method for treatment of a disease, and thus do not comply with PCT Rule 39.1(iv). In this report, a search is carried out on the basis of the solution in which the subject matter thereof is amended to be a pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/142028**

| Patent document cited in search report | | | Publication date (day/month/year) | Patent family member(s) | | | Publication date (day/month/year) |
|---|---|---|---|---|---|---|---|
| CN | 116041518 | A | 02 May 2023 | None | | | |
| CN | 112961242 | A | 15 June 2021 | None | | | |
| CN | 111944050 | A | 17 November 2020 | None | | | |
| CN | 103687945 | A | 26 March 2014 | HUE | 038685 | T2 | 28 November 2018 |
| | | | | US | 2013078234 | A1 | 28 March 2013 |
| | | | | US | 9371395 | B2 | 21 June 2016 |
| | | | | CA | 2834136 | A1 | 01 November 2012 |
| | | | | CA | 2834136 | C | 17 April 2018 |
| | | | | ES | 2667568 | T3 | 11 May 2018 |
| | | | | MY | 173377 | A | 21 January 2020 |
| | | | | JPWO | 2012147713 | A1 | 28 July 2014 |
| | | | | JP | 5917498 | B2 | 18 May 2016 |
| | | | | CO | 6811812 | A2 | 16 December 2013 |
| | | | | EP | 2703486 | A1 | 05 March 2014 |
| | | | | EP | 2703486 | A4 | 25 February 2015 |
| | | | | EP | 2703486 | B1 | 07 March 2018 |
| | | | | PT | 2703486 | T | 18 May 2018 |
| | | | | ZA | 201307983 | B | 27 January 2016 |
| | | | | JP | 2016165294 | A | 15 September 2016 |
| | | | | JP | 6224759 | B2 | 01 November 2017 |
| | | | | TW | 201249869 | A | 16 December 2012 |
| | | | | TWI | 561531 | B | 11 December 2016 |
| | | | | DK | 2703486 | T3 | 28 May 2018 |
| | | | | RU | 2013152164 | A | 27 May 2015 |
| | | | | RU | 2668170 | C2 | 26 September 2018 |
| | | | | KR | 20140033018 | A | 17 March 2014 |
| | | | | KR | 102030987 | B1 | 11 November 2019 |
| | | | | MX | 2013012285 | A | 21 November 2013 |
| | | | | MX | 344773 | B | 06 January 2017 |
| | | | | SG | 194620 | A1 | 30 December 2013 |
| | | | | IL | 229061 | A0 | 31 December 2013 |
| | | | | IL | 229061 | B | 28 February 2019 |
| | | | | SI | 2703486 | T1 | 31 May 2018 |
| | | | | HRP | 20180640 | T1 | 01 June 2018 |
| | | | | TR | 201808018 | T4 | 21 June 2018 |
| | | | | PL | 2703486 | T3 | 31 July 2018 |
| | | | | US | 2016368990 | A1 | 22 December 2016 |
| | | | | RS | 57279 | B1 | 31 August 2018 |
| | | | | LT | 2703486 | T | 25 May 2018 |
| | | | | NZ | 616809 | A | 28 August 2015 |
| | | | | AU | 2012248470 | A1 | 21 November 2013 |
| | | | | AU | 2012248470 | B2 | 27 October 2016 |
| | | | | WO | 2012147713 | A1 | 01 November 2012 |
| CN | 109851673 | A | 07 June 2019 | WO | 2020151384 | A1 | 30 July 2020 |
| CN | 115066436 | A | 16 September 2022 | CA | 3161818 | A1 | 27 May 2021 |
| | | | | EP | 3822288 | A1 | 19 May 2021 |
| | | | | EP | 4061841 | A1 | 28 September 2022 |
| | | | | US | 2023007977 | A1 | 12 January 2023 |
| | | | | KR | 20220100693 | A | 15 July 2022 |

Form PCT/ISA/210 (patent family annex) (July 2022)

**INTERNATIONAL SEARCH REPORT**
Information on patent family members

| | International application No. |
| --- | --- |
| | **PCT/CN2023/142028** |

| Patent document cited in search report | Publication date (day/month/year) | Patent family member(s) | | | Publication date (day/month/year) |
| --- | --- | --- | --- | --- | --- |
| | | JP | 2023511482 | A | 20 March 2023 |
| | | AU | 2020389038 | A1 | 26 May 2022 |
| | | WO | 2021099347 | A1 | 27 May 2021 |
| WO 2021027674 A1 | 18 February 2021 | None | | | |

Form PCT/ISA/210 (patent family annex) (July 2022)

**EP 4 644 420 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 202211681398 A

**Non-patent literature cited in the description**

- **KONTOS, F. et al.** B7-H3: An Attractive Target for Antibody-based Immunotherapy.. *Clin Cancer Res*, 2021, vol. 27 (5), 1227-1235
- **LEE, Y.-H. et al.** Inhibition of the B7-H3 immune checkpoint limits tumor growth by enhancing cytotoxic lymphocyte function. *Cell research*, 2017, vol. 27 (8), 1034-1045
- **CAI, D et al.** Tumor-expressed B7-H3 mediates the inhibition of antitumor T-cell functions in ovarian cancer insensitive to PD-1 blockade therapy. *Cell Mol Immunol*, 2020, vol. 17 (3), 227-236
- **LEE, Y.H. et al.** Inhibition of the B7-H3 immune checkpoint limits tumor growth by enhancing cytotoxic lymphocyte function. *Cell Res*, 2017, vol. 27 (8), 1034-1045
- **LU, H. et al.** B7-H3 inhibits the IFN-gamma-dependent cytotoxicity of Vgamma9Vdelta2 T cells against colon cancer cells.. *Oncoimmunology*, 2020, vol. 9 (1), 1748991
- **SCRIBNER, J.A. et al.** Preclinical Development of MGC018, a Duocarmycin-based Antibody-drug Conjugate Targeting B7-H3 for Solid Cancer.. *Mol Cancer Ther*, 2020, vol. 19 (11), 2235-2244
- **LOO, D. et al.** Development of an Fc-enhanced anti-B7-H3 monoclonal antibody with potent antitumor activity.. *Clin Cancer Res*, 2012, vol. 18 (14), 3834-45

- **VALLERA, D.A. et al.** NK-Cell-Mediated Targeting of Various Solid Tumors Using a B7-H3 Tri-Specific Killer Engager In Vitro and In Vivo.. *Cancers*, 2020, vol. 12 (9)
- **TANG, X. et al.** Administration of B7-H3 targeted chimeric antigen receptor-T cells induce regression of glioblastoma.. *Signal Transduct Target Ther*, 2021, vol. 6 (1), 125
- **THERUVATH, J et al.** Locoregionally administered B7-H3-targeted CAR T cells for treatment of atypical teratoid/rhabdoid tumors.. *Nat Med*, 2020, vol. 26 (5), 712-719
- **TANG, X. et al.** Bioactivity and safety of B7-H3-targeted chimeric antigen receptor T cells against anaplastic meningioma.. *Clinical & Translational Immunology*, 2020, vol. 9 (6), e1137
- **ANG, W.X. et al.** Electroporation of NKG2D RNA CAR Improves Vγ9Vδ2 T Cell Responses against Human Solid Tumor Xenografts.. *Molecular Therapy - Oncolytics*, 2020, vol. 17, 421-430
- **ROZENBAUM, M. et al.** Gamma-Delta CAR-T Cells Show CAR-Directed and Independent Activity Against Leukemia.. *Frontiers in Immunology*, 2020, vol. 11
- Sambrook Molecular Cloning: A Laboratory Manual. Cold Spring Harbor Laboratory, 1989

43